# EUROPEAN PATENT APPLICATION

(11) **EP 2 662 085 A1**
(43) Date of publication of application: **13.11.2013**
(21) Application number: 12167693.6
(22) Date of filing: 11.05.2012
(51) Int. Cl.: A61K 35/14, C12N 5/0786

(54) **Regulatory Macrophages and Their Uses**

(71) Applicant: Humboldt-Universität zu Berlin, 10099 Berlin (DE)
(72) Inventor: The designation of the inventor has not yet been filed
(74) Representative: Engelhard, Markus

(57) **Abstract**

The present invention relates to regulatory monocytes and regulatory macrophages and their uses.

## Description

The present invention relates to regulatory macrophages and their uses.

Atopic and autoimmune diseases are characterized by undesirable inflammation that contributes to less well-being and life expectancy of human beings. Atopic diseases encompass a wide range of immunologic disorders, e. g. hay fever, atopic dermatitis, allergic sinusitis, food allergies and asthma. Among these, especially allergic asthma is a frequently occurring phenomenon in westernized countries. It affects 200 to 300 million people worldwide (Fallon and Mangan, 2007) and is triggered by innocuous antigens that are usually well tolerated. At present glucocorticoides and antihistamines are most commonly used to treat allergy-related disorders. The disadvantages of this therapy are side effects like osteoporosis, the need to take the drugs life-long and an unsatisfactory disease control in 30% of allergic patients (Hawrylowicz and O'Garra, 2005). In addition, not only the incidence of atopic diseases rose dramatically in westernized countries, also Th1-biased autoimmune diseases, e. g. multiple sclerosis, type 1 diabetes or morbus crohn, considerably increased during the last decades (Yazdanbakhsh et al., 2002; Bach, 2002). Such autoimmune diseases are generally treated with immunosuppressant drugs, e. g. corticosteroids which, in the long run, can lead to diabetes, eye disease (glaucoma or cataract), stomach ulcers, high blood pressure or depression. Thus, alternative treatment strategies are urgently required.

Helminth parasites are eukaryotic organisms which live as extracellular parasites in other organisms to obtain nourishment thereby inflicting morbidity. In recent years, epidemiological and laboratory studies, however, showed that helminth parasites modulate the host immune system to ensure their survival and thereby exert spillover effects on unrelated immune dysfunctions. Furthermore, successful treatment of inflammatory bowel or Crolin's s disease patients with *Trichuris suis* or *Necator americanus* in clinical trials demonstrated that helminth worms can be used for therapeutic purposes (Summers et al. 2005; Croese et al., 2006). However, although the concept of helminthic therapy has been described as safe and effective, the application of living parasites always bears the risk of unwanted side effects which might be prevented by using alternative strategies, e. g. single helminth regulatory products.

Recently, patent application PCT/EP2007/006888 (published as WO 2008/015014 A2) has presented a novel approach to treat inflammatory diseases: by treating a patient with the protein cystatin derived from nematodes, inflammatory immune reactions can be suppressed. This way, for example the induction of allergic airway hyperreactivity, or the induction of allergic gastrointestinal hyperreactivity can be inhibited.

However, although therapeutic strategies using helminth-derived immunomodulators are more purposive than the treatment with living worms, the big disadvantage is the immunogenic activity of some molecules. Nematode cystatin is an immunogenic molecule and is recognized by the immune system of the patient. Accordingly, a patient treated with cystatin may develop immune reactions against the molecule, leading to undesired side effects. A patient sensitized to cystatin by a previous cystatin treatment may react to another exposure with an immunologic overreaction that may even take the form of an allergic shock. Alternatively, if cystatin treatment is repeated later on, the intended effects may be significantly reduced, because the patient's immune system neutralizes the cystatin molecule immediately upon entry into the patient's body.

Moreover, the approach of PCT/EP2007/006888 requires repeated administration of cystatin to the patient, meaning an increased effort on side of the patient and the risk that the therapy may fail due to deviations from the treatment schedule.

Accordingly, there exists a need in the art to provide for improved ways of therapy for inflammatory diseases.

It is therefore one object of the present invention to provide for improved ways to prevent and/or treat inflammatory diseases, in particular atopic and/or autoimmune diseases. Moreover, it is an object of the present invention to provide for ways of preventing and/or treating inflammatory diseases that do not elicit inflammatory immune reactions of a treated patient against any of the substances administered in connection with the treatment. Furthermore, it is an object of the present invention to provide for ways of preventing and/or treating inflammatory diseases that do not become less effective when they are repeated later on. Furthermore, it is an object of the present invention to provide for ways of preventing and/or treating inflammatory diseases that do not require repeated administration to the patient.

The objects of the present invention are solved by a macrophage cell for use in the prevention and/or treatment of an inflammatory disease in a patient,
wherein, preferably, said cell has been produced by treatment with a cystatin derived from a parasitic nematode and
wherein said cell differs from murine macrophages of subpopulation M2a (by a higher expression level of the gene SPHK1 and/or the gene LIGHT,) and from murine macrophages of subpopulation M2b (by a higher expression level of the gene ARG1,) as measured by real-time PCR.

Sometimes as used herein, the above-described characteristics of said cell, i.e. that said cell differs from murine macrophages of subpopulation M2a (by a higher expression level of the gene SPHK1 and/or the gene LIGHT,) and from murine macrophages of subpopulation M2b (by a higher expression level of the gene ARG1,) as measured by real-time PCR, is referred to as a "hybrid phenotype".

In a preferred embodiment, said cell is an isolated cell, preferably an isolated monocyte cell or an isolated macrophage cell.

Preferably, such measurement is carried out at a time point that lies in the range of from 6 h to 7 days, preferably at 6 h, 12 h, 24 h, 36 h, 48 h, 3 days, 4, days, 5 days, 6 days, or 7 days after said treatment with a cystatin derived from a parasitic nematode.

In one embodiment, said treatment with said cystatin derived from a parasitic nematode occurs *in vivo.* In one embodiment, said treatment with said cystatin derived from a parasitic nematode occurs *in vitro,* preferably in the presence of bystander cells, more preferably in the presence of leukocytes, which are removed after the treatment.

In one embodiment, said macrophage cell is an autologous cell with respect to said patient.

In one embodiment, said macrophage cell is administered to said patient, preferably by intravenous administration, more preferably by intravenous injection.

In one embodiment, after said cell is produced and before said cell is administered to said patient, said cell is stored in a frozen state.

In one embodiment, said cell differs from macrophages of subpopulation M2a by a lower expression level of the genes Ym1 and/or RELM-α, as measured by real-time PCR, wherein, preferably, said lower expression level of Ym1 and/or RELM-α is, independently, at least 2-fold, preferably at least 5-fold, more preferably at least 10-fold, even more preferably at least 20-fold, most preferably at least 50-fold.

Preferably, such measurement is carried out at a time point that lies in the range of from 6 h to 7 days, preferably at 6 h, 12 h, 24 h, 36 h, 48 h, 3 days, 4, days, 5 days, 6 days, or 7 days after said treatment with a cystatin derived from a parasitic nematode.

In one embodiment, said higher expression level of SPHK1, LIGHT, and/or ARG1 is, independently, at least 2-fold, preferably at least 5-fold, more preferably at least about 10-fold, more preferably at least about 20-fold, more preferably at least about 30-fold, more preferably at least about 40-fold, more preferably at least 50-fold, most preferably at least 100-fold.

In one embodiment, said inflammatory disease is an atopic and/or autoimmune disease, preferably selected from the group comprising allergic diseases of the respiratory organs, such as asthma, hay fever, allergic sinusitis, allergic rhinitis, more preferably asthma or hay fever, allergic diseases of the gastrointestinal system, such as food allergies, allergic diseases of the skin, such as atopic dermatitis, systemic allergic diseases, such as anaphylactic reactions, allergic encephalomyelitis, autoimmune diseases of the joints and/or skin and/or internal organs, such as rheumatoid arthritis, psoriasis, lupus erythematosus, multiple sclerosis, and inflammatory bowel diseases, such as colitis ulcerosa and Crohn's Disease.

In one embodiment, said inflammatory bowel disease is colitis, preferably colitis ulcerosa. The term "colitis", as used herein, is meant to include both acute and chronic colitis.

In one embodiment, said cell is a cell for use in the prevention of a seasonal allergy, preferably a cell for use in the prevention of hay fever.

In one embodiment, said cell is part of a mixture of cells with a content of macrophages higher than 85%, preferably higher than 90%, more preferably higher than 95%, most preferably higher than 98%.

In one embodiment, said cystatin has a sequence selected from the group comprising SEQ ID NO:1 (*Acanthocheilonema viteae* cystatin L43053), SEQ ID NO: 2 (*Onchocerca volvulus* cystatin M37105), SEQ ID NO:3 (*Brugia malayi* cystatin), and sequences that are 70% identical, preferably 80% identical, more preferably 90% identical and, most preferably, 99% identical to any of the foregoing.

In one embodiment, said cystatin is recombinant cystatin and has been produced by a prokaryotic or eukaryotic expression system.

In one embodiment, said disease is associated with an increased count of eosinophil blood cells and/or with an increased level of IgE, when compared with a patient not having said disease, and said medicament, upon its administration to said patient, leads to a reduction of said increased count of eosinophil blood cells and/or to a reduction of said increased level of IgE, preferably to a count of eosinophil blood cells and/or to a level of IgE of a healthy individual.

In one embodiment, said increased count of eosinophil blood cells is > 4% of all white blood cells of a patient or > 360/µl total number of eosinophil blood cells in peripheral blood of a patient, and said increased level of IgE is > 100 kU/l serum level in an adult patient.

In one embodiment, said patient is a mammal, preferably a human being.

In one embodiment, said patient is an animal, preferably selected from the group comprising a canine animal, preferably a dog, a feline animal, preferably a cat, and a rodent, preferably a mouse.

In one embodiment, said cell is administered to said patient by irrigation.

In one embodiment, said administration occurs less than three times, preferably only once, per occurrence of said disease in said patient.

In one embodiment, said parasitic nematode is parasitic to humans or animals.
The term "animal", as used herein, refers to non-human animals.

In one embodiment, said parasitic nematode is parasitic to canine animals, preferably dogs, or is parasitic to rodents, preferably mice, or is parasitic to feline animals, preferably cats.

In one embodiment, said nematode is selected from the group comprising *Onchocerca volvulus, Brugia malayi, Wuchereria bancrofti, Loa loa* and *Acanthocheilonema viteae, Dirofilaria immitis, Dirofilaria repens, Nippostrongylus brasiliensis* and *Litomosoides sigmodontis.*

In one embodiment, said cell is administered to said patient in combination with one or more drugs selected from the group of anti-inflammatory drugs, preferably corticosteroids, non-steroidal anti-inflammatory drugs, and/or anti-histamines.

In one embodiment, said administration of said cell to said patient occurs in combination with another therapeutic treatment of said patient that is aimed at treating the same inflammatory disease as treated by administration of said cell.

In one embodiment, said cell differs from macrophages that have not been produced by treatment with a cystatin derived from a parasitic nematode by an increased expression, preferably by a factor of 10, of the cell surface protein MHC-II, and/or by an increased expression, preferably by a factor of 10, of the cell surface protein CD40, and/or by an increased expression, preferably by a factor of 10, of the cell surface protein PDL-2, as measured by antibody staining of cell surface proteins and flow cytometric analysis.

Preferably, such measurement is carried out at a time point that lies in the range of from 6 h to 7 days, preferably at 6 h, 12 h, 24 h, 36 h, 48 h, 3 days, 4, days, 5 days, 6 days, or 7 days after said treatment with a cystatin derived from a parasitic nematode.

In one embodiment, said cell induces T cells, preferably CD4+ T cells, to produce IL-10.

The objects of the present invention are also solved by an autologous macrophage cell for use in the prevention and/or treatment of an inflammatory disease in a patient, wherein said macrophage cell is prepared by the steps:
(a)
   - obtaining a macrophage cell derived from the body of said patient,
   - activating said macrophage cell *in vitro* by treatment with a cystatin derived from a parasitic nematode, wherein, preferably, said cell is activated in the presence of bystander cells, preferably leukocytes, which, preferably, are removed after the *in vitro* treatment,
      or
(b)
   - activating a macrophage cell in the body of said patient,
   - obtaining said macrophage cell from the body of said patient.

In one embodiment, after said macrophage cell has been prepared, it is administered to said patient, preferably by intravenous administration, more preferably by intravenous injection.

In one embodiment, after said cell is prepared and before said cell is administered to said patient, said cell is stored in a frozen state.

In one embodiment, said obtaining said cell derived from the body of said patient involves one of the following:
A) collecting blood from said patient and isolating macrophage-precursors (monocytes) from said blood, preferably by differential adhesion to tissue culture plastic, optionally after removing components of the blood that are not cells, and differentiating said macrophage-precursors into macrophages in vitro by incubating said cells with macrophage colony-stimulating factor (M-CSF).
B) collecting blood of said patient and removing cell populations other than macrophage precursors from said blood, preferably removing T cells, B cells, granulocytes, dendritic cells, eosinophiles and/or NK cells, optionally after removing components of the blood that are not cells, and differentiating said macrophage-precursors into macrophages in vitro by incubating said cells with macrophage colony-stimulating factor (M-CSF) wherein, preferably, said removing of cell populations other than macrophage precursors is achieved by cell depletion with antibodies binding to cell surface molecules of the respective cell types,
C) obtaining peritoneal exudate cells and, optionally, removing cell populations other than macrophages, preferably removing T cells, B cells, granulocytes, dendritic cells, eosinophiles and/or NK cells, preferably by cell depletion with antibodies binding to cell surface molecules of the respective cell types,
D) generating macrophages from induced human Pluripotent stem cells (iPS) of said patients (Senju et al. 2011),
   In one embodiment, said cell differs from murine macrophages of subpopulation M2a by a higher expression level of the genes SPHK1 and/or LIGHT, as measured by real-time PCR, and/or wherein said cell differs from murine macrophages of subpopulation M2b by a higher expression level of the gene ARG1, as measured by real-time PCR. Preferably, such measurement is carried out at a time point that lies in the range of from 6 h to 7 days, preferably at 6 h, 12 h, 24 h, 36 h, 48 h, 3 days, 4, days, 5 days, 6 days, or 7 days after said treatment with a cystatin derived from a parasitic nematode.

In one embodiment, said cell differs from murine macrophages of subpopulation M2a by a lower expression level of the genes Ym1 and/or RELM-α, as measured by real-time PCR, wherein, preferably, said lower expression level of Ym1 and/or RELM-α is, independently, at least 2-fold, preferably at least 5-fold, more preferably at least 10-fold, even more preferably at least 20-fold, most preferably at least 50-fold.

Preferably, such measurement is carried out at a time point that lies in the range of from 6 h to 7 days, preferably at 6 h, 12 h, 24 h, 36 h, 48 h, 3 days, 4, days, 5 days, 6 days, or 7 days after said treatment with a cystatin derived from a parasitic nematode.

In one embodiment, said higher expression level of SPHK1, LIGHT, and/or ARG1 is, independently, at least 2-fold, preferably at least 5-fold, more preferably at least about 10-fold, more preferably at least about 20-fold, more preferably at least about 30-fold, more preferably at least about 40-fold, more preferably at least 50-fold, most preferably at least 100-fold.

In one embodiment, said inflammatory disease is an atopic and/or autoimmune disease, preferably selected from the group comprising allergic diseases of the respiratory organs, such as asthma, hay fever, allergic sinusitis, allergic rhinitis, more preferably asthma or hay fever, allergic diseases of the gastrointestinal system, such as food allergies, allergic diseases of the skin, such as atopic dermatitis, systemic allergic diseases, such as anaphylactic reactions, allergic encephalomyelitis, autoimmune diseases of the joints and/or skin and/or internal organs, such as rheumatoid arthritis, psoriasis, lupus erythematosus, multiple sclerosis, and inflammatory bowel diseases, such as colitis ulcerosa and Crohn's Disease.

In one embodiment, said inflammatory bowel disease is colitis, preferably colitis ulcerosa. The term "colitis", as used herein, is meant to include both acute and chronic colitis.

In one embodiment, said cell is part of a mixture of cells with a content of macrophages higher than 85%, preferably higher than 90%, more preferably higher than 95%, most preferably higher than 98%.

In one embodiment, said cystatin has a sequence selected from the group comprising SEQ ID NO:1 *(Acanthocheilonema viteae* cystatin L43053), SEQ ID NO: 2 (*Onchocerca volvulus* cystatin M37105), SEQ ID NO:3 (*Brugia malayi* cystatin), and sequences that are 70% identical, preferably 80% identical, more preferably 90% identical and, most preferably, 99% identical to any of the foregoing.

In one embodiment, said cystatin is recombinant cystatin and has been produced by a prokaryotic or eukaryotic expression system.

In one embodiment, said disease is associated with an increased count of eosinophil blood cells and/or with an increased level of IgE, when compared with a patient not having said disease, and said medicament, upon its administration to said patient, leads to a reduction of said increased count of eosinophil blood cells and/or to a reduction of said increased level of IgE, preferably to a count of eosinophil blood cells and/or to a level of IgE of a healthy individual.

In one embodiment, said increased count of eosinophil blood cells is > 4% of all white blood cells of a patient or > 360/µl total number of eosinophil blood cells in peripheral blood of a patient, and said increased level of IgE is > 100 kU/l serum level in an adult patient.

In one embodiment, said patient is a mammal, preferably a human being.

In one embodiment, said patient is an animal, preferably selected from the group comprising a canine animal, preferably a dog, a feline animal, preferably a cat, and a rodent, preferably a mouse.

In one embodiment, said administration occurs less than three times, preferably only once, per occurrence of said disease in said patient.

In one embodiment, said parasitic nematode is parasitic to humans or animals.
The term "animal", as used herein, refers to non-human animals.

In one embodiment, said parasitic nematode is parasitic to canine animals, preferably dogs, or is parasitic to rodents, preferably mice, or is parasitic to feline animals, preferably cats.

In one embodiment, said nematode is selected from the group comprising *Onchocerca volvulus, Brugia malayi, Wuchereria bancrofti, Loa loa* and *Acanthocheilonema viteae, Dirofilaria immitis, Dirofilaria repens, Nippostrongylus brasiliensis* and *Litomosoides sigmodontis.*

In one embodiment, said cell is administered to said patient in combination with one or more drugs selected from the group of anti-inflammatory drugs, preferably corticosteroids, non-steroidal anti-inflammatory drugs, and/or anti-histamines.

In one embodiment, said administration of said cell to said patient occurs in combination with another therapeutic treatment of said patient that is aimed at treating the same inflammatory disease as treated by administration of said cell.

In one embodiment, said cell differs from macrophages that have not been treated with a cystatin derived from a parasitic nematode by an increased expression, preferably by a factor of 10, of the cell surface protein MHC-II, and/or by an increased expression, preferably by a factor of 10, of the cell surface protein CD40, and/or by an increased expression, preferably by a factor of 10, of the cell surface protein PDL-2, as measured by antibody staining of cell surface proteins and flow cytometric analysis.

Preferably, such measurement is carried out at a time point that lies in the range of from 6 h to 7 days, preferably at 6 h, 12 h, 24 h, 36 h, 48 h, 3 days, 4, days, 5 days, 6 days, or 7 days after said treatment with a cystatin derived from a parasitic nematode.

In one embodiment, said cell induces T cells, preferably CD4+ T cells, to produce IL-10.

The objects of the present invention are also solved by a method for prevention and/or treatment of an inflammatory disease in a patient, said method comprising the steps:
- obtaining macrophage precursor cells (monocytes, iPS) derived from the body of said patient,
- differentiating said precursor cells into macrophages by M-CSF
- activating said macrophage cells *in vitro* by treatment with cystatin derived from a parasitic nematode
- administering said macrophage cells to said patient.

In such method, said inflammatory disease, said patient, said obtaining macrophage cells, said activating said macrophage cells, said cystatin, said parasitic nematode, and said administering are as defined in the embodiments above.

The objects of the present invention are also solved by a method of using the macrophage cell as described above for treatment of an inflammatory disease in a patient.

In such method, said inflammatory disease, said patient and said macrophage cell are as defined in the embodiments above.

The objects of the present invention are also solved by the use of a macrophage cell for the manufacture of a medicament for the treatment of an inflammatory disease in a patient.

In such use, said inflammatory disease, said patient, and said macrophage cell are as defined in the embodiments above.

As used herein, the term "cystatin" refers to members of a super family of inhibitors of cysteine proteases.

A "parasitic nematode" is a nematode that exploits a host organism for meeting its own requirements. This may involve a life of the nematode within the tissue of a host for parts or the entire life-span of the nematode. In preferred embodiments, the host of such parasitic nematode is human.

A sequence that has x% identity to another sequence is a sequence in which x% residues on their respective positions are identical when optimally aligned.

The term "a cystatin derived from a nematode" as used herein, is meant to refer to any cystatin that has the sequence of a cystatin occurring in a nematode. The term is not limited to a specific production method and may include isolation of the cystatin from the nematode or production of the cystatin by other methods, such as recombinant techniques or chemical synthesis. The term "a cystatin derived from a nematode" is also meant to include proteins that have retained a cystatin function despite their amino acid sequence having been mutated in one or several positions by substitutions, insertions or deletions. Techniques for producing such mutated cystatins which nevertheless can be considered as being "derived from a nematode" have been described in, for example, "Proteins, Structures and Molecular Properties" by Thomas E. Creighton, second edition, W. H. Freeman and Co., New York, and are known to someone skilled in the art.

The term "said cell is administered to said patient in combination with another drug" is meant to include any situation wherein said cell is administered concomitantly with, before or after administration of such another drug. The other drug and said cell may be in the same dosage unit, or they may be administered in separate dosage units. They may be administered by the same route or different routes.

The expression that the "administration of said cell to said patient occurs in combination with another therapeutic treatment" is meant to include any situation wherein said cell is administered to said patient previous to, simultaneously with, subsequent to, or overlapping with said other therapeutic treatment.

As used herein, the term "autologous cell" refers to a situation in which said cell is derived from the same patient to whom the cell is administered later on.

The terms "macrophage cell" and "macrophage" are used interchangeably and refer to a monocyte-derived phagocyte which is not a dendritic cell or a cell that derives from tissue macrophages by local proliferation. In the body these cells are tissue specific and refer to e. g. Kupffer cells in the liver, alveolar macrophages in the lung, microglia cells in the brain, osteoclasts in the bone etc. The skilled person is aware how to identify macrophage cells, how to isolate macrophage cells from the body of a human or animal, and how to characterize macrophage cells with respect to their subclass and subpopulation (Kruisbeek, 2001; Davies and Gordon 2005 a and b; Zhang et al., 2008; Mosser and Zhang, 2008; Weischenfeldt and Porse, 2008; Ray and Dittel, 2010; Martinez et al., 2008; Jenkins et al., 2011).

Murine macrophages can be polarized, e. g. differentiated into specific subclasses and subpopulations, such as the subclasses designated M1 and M2 macrophages (Mills et al., 2000). Whereas the term M1 is used to describe classically activated macrophages that arise due to injury or bacterial infection and IFN-y activation, M2 has been introduced as a generic term for numerous forms of macrophages activated differently than M1. The M2 classification has further been divided into subpopulations (Mantovani et al., 2004). The most representative form is M2a macrophages, which commonly occur in helminth infections by exposure to worm induced Th2 cytokines IL-4 and IL-13. M2a macrophages were, among others, shown to be essentially involved in protecting the host from re-infection (Anthony et al., 2006) or in contributing to wound healing and tissue remodeling (Gordon, 2003). Another subpopulation is M2b macrophages that produce high levels of IL-10 and low levels of IL-12 but are not per se anti-inflammatory (Anderson and Mosser, 2002; Edwards et al., 2006). M2b macrophages are elicited by immune complexes that bind to Fc-γ receptors in combination with TLR ligands. Finally, M2c macrophages represent a subtype elicited by IL-10, TGF-β or glucocorticoids and can be regarded as deactivated macrophages (Martinez et al., 2008).

If the present application refers to "macrophages of subpopulation M2a", this is meant to designate a murine macrophage cell that has been exposed to a milieu under Th2 conditions (e g. exposure to Th2 cytokines IL-4 and IL-13) and exhibits a specific phenotype by higher expression of the gene Ym1 and/or the gene CD206 and/or the gene RELM-α and/or the gene Arginase-1. Said M2a macrophages are less efficient in antigen-presentation and lack the ability to produce nitric oxide thus showing impaired microbicidal activity.
If the present application refers to "macrophages of subpopulation M2b", this is meant to designate a murine macrophage cell that has been exposed to a milieu of immune complexes in combination with TLR or TNF-alpha stimulation. Said cell is characterized through higher expression of the gene SPHK-1 and/or the gene LIGHT and/or the gene IL-10.

At some instances, the present application refers to a macrophage cell "derived from the body of a patient". This is meant to designate that either macrophages are obtained from the body of said patient, or macrophage precursor cells are obtained from the body of said patient and subsequently differentiated into macrophage cells *in vitro* as described in Wahl et al. 2006; Davis and Gordon 2005; Smythies et al., 2006; Zhang et al., 2008; Mosser and Zhang, 2008.

The term "obtaining a cell", as used herein, is meant to include both obtaining of said cell in pure form and obtaining a cell mixture that contains said cell.

The phrase "a cell has been produced by treatment with cystatin" is meant to refer to a situation where either with the cell or a precursor of said cell or an organism in which said cell or a precursor of said cell resided at the time has been subjected to cystatin treatment.

The term "activating" a macrophage cell refers to a process in which the macrophage cell is subjected to a treatment that results in changes to the characteristics of said macrophage cell, such as changes in the expression of certain marker genes.

The term *"in vitro* ", as used herein, refers to a situation where something takes place outside of an organism. If used in connection with cells being subjected to a treatment, such cells may be either in culture or not, they may have been outside of an organism for any length of time, and they may remain outside of an organism after the treatment for any length of time.

The term "bystander cells", as used herein, refers to all cell types other than macrophages.

The term "isolating" macrophage-precursors, e.g. from blood, refers to a situation where the macrophage-precursors are being enriched compared to other types of cells. The enrichment may be to a purity of higher than 85%, preferably higher than 90%, more preferably higher than 95%, most preferably higher than 98%, wherein these percentages refer to the number of macrophage-precursors compared to the total number of cells.

When the present application speaks of isolating macrophage-precursors "by differential adhesion to tissue culture plastic", this refers to a situation where macrophages are isolated by exploiting that there are differences in the adhesion of different cell types to the plastic surface of a tissue culture dish. Specifically, this may involve culture of a mixture of different types of cells in a tissue culture dish at conditions where only some types of cells adhere to the plastic surface of a tissue culture dish. By separating the cells that adhere to the tissue culture dish from the cells that do not adhere, an enrichment for macrophage-precursors can be achieved.

The term "expression", as used herein, refers to the process by which the information encoded in a DNA sequence is first converted into an RNA sequence (transcription), and then further into a protein sequence (translation). The term is used to refer to either the transcription step or the translation step individually, or both steps together. A "higher expression level" of a certain gene, as used herein, is meant to designate that one cell type or cellular situation differs from another cell type or cellular situation by having, due to an increased expression of that gene, an increased level of the RNA and/or the protein encoded by that gene. Such an increased expression level can for example be detected by real-time PCR, if an increased level of an RNA is to be detected, or, if the encoded protein is a cell surface protein, by antibody staining of the cell surface protein and flow cytometric analysis.

The terms "SPHK1 ", "LIGHT", "ARG1", "Ym1", RELM-α are gene designations for mouse genes.

The term "inflammatory disease", as used herein, refers to diseases which are characterized by the presence of undesirable inflammation and inflammatory symptoms. The term includes both atopic and autoimmune diseases. Examples of inflammatory diseases in the sense of the present application are allergic diseases of the respiratory organs, such as asthma, hay fever, allergic sinusitis, allergic rhinitis, of the gastrointestinal system, such as food allergies, of the skin, such as atopic dermatitis, systemic allergic diseases, such as anaphylactic reactions, allergic encephalomyelitis, autoimmune diseases of the joints and/or skin and/or internal organs, such as rheumatoid arthritis, psoriasis, lupus erythematosus, multiple sclerosis, and inflammatory bowel diseases, such as colitis ulcerosa and Crohn's Disease.

The present inventors have now surprisingly found that treatment of a patient with cystatin derived from parasitic nematodes results in the formation of a special population of regulatory macrophages that have immunosuppressive function and are characterized by a specific expression pattern of certain marker genes. A population of regulatory macrophages with the same characteristics is formed when macrophages are treated with cystatin derived from nematodes *in vitro.* Macrophages of this population are sufficient to suppress major symptoms of allergic airway disease and colitis. The immunoregulatory function of the suppressive macrophage is conveyed by a soluble factor and is associated with the induction of IL-10 producing T cells.

In the following, reference is made to the figures:

### Figure 1

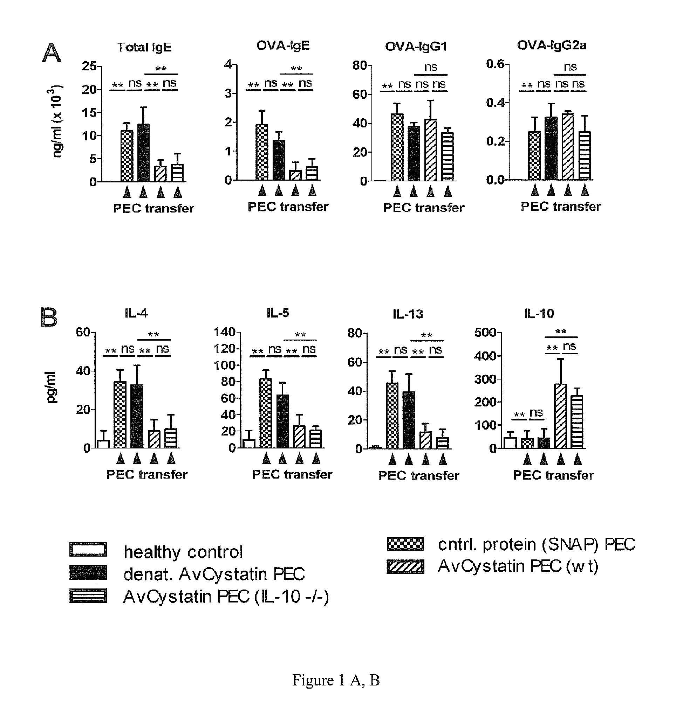

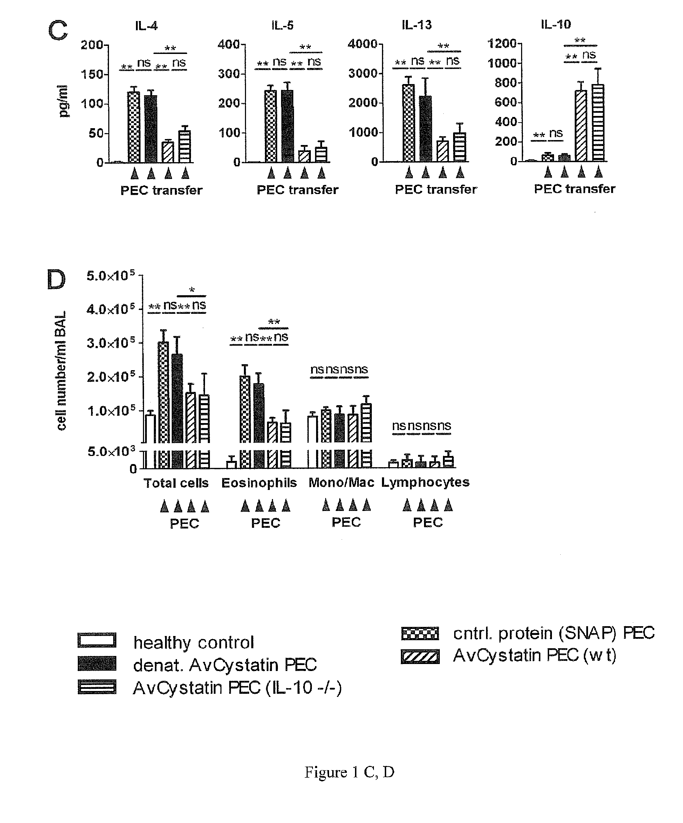

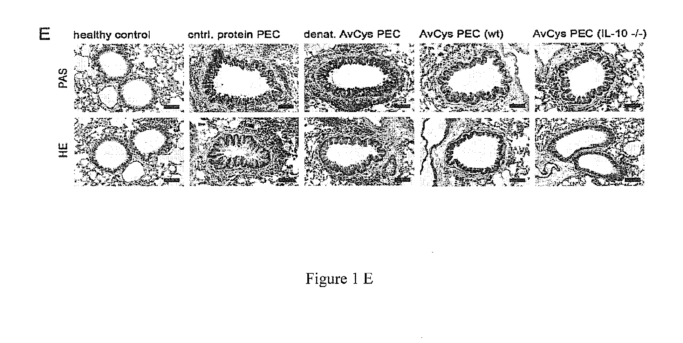

Figure 1 shows that transfer of AvCystatin *(Acanthocheilonema viteae* cystatin) treated peritoneal exudate cells (PEC) from wildtype and IL-10 deficient mice protects against ovalbumin-induced airway inflammation.

Whole peritoneal exudate cells were isolated from AvCystatin and control (denatured AvCystatin or SNAP-tag protein (New England Biolabs) pre-treated wildtype or IL-10 deficient mice 18 hours post injection and adoptively transferred in ovalbumin (OVA) sensitized mice.

Panel A shows the detection of total and allergen-specific levels of serum IgE or OVA-specific IgG1 and IgG2a in recipient mice.

Panel B shows the local cytokine response in BAL (bronchioalveolar lavage)-fluid measured by quantification of IL-4, IL-5, IL-13 and IL-10 by ELISA (enzyme-linked immunosorbent assay).

Panel C shows the systemic cytokine response in culture supernatants of allergen restimulated splenocytes.

Panel D shows cell counts for inflammatory cells in BAL-fluid.

Panel E shows histological stainings of representative lung bronchiole sections with periodic acid schiff (PAS) and hematoxylin eosin (HE). The samples "AvCys PEC (wildtype)" and "AvCys PEC (IL-10 -/-)" show reduced levels of PAS and HE staining around the lumen compared to the control samples "cntrl. protein PEC" and "denat. AvCys PEC". Thus, the figure illustrates reduced mucus production (PAS staining) and reduced infiltration of inflammatory cells (HE staining) into the airways of asthmatic mice (Ova model) after injection of AvCystatin primed peritoneal exudate cells (PEC) from wild type mice or IL-10 deficient mice. Transfer of AvCystatin primed PEC into asthmatic mice is able to soften asthma symptoms. The effect is not mediated by IL-10 derived from primary primed AvCystatin PEC. Bars, 100 µm.

The results are presented as means ± SEM (standard error of the mean) from two independent experiments with 5-6 animals per group. *, P < 0.05; **, P < 0.01.

### Figure 2

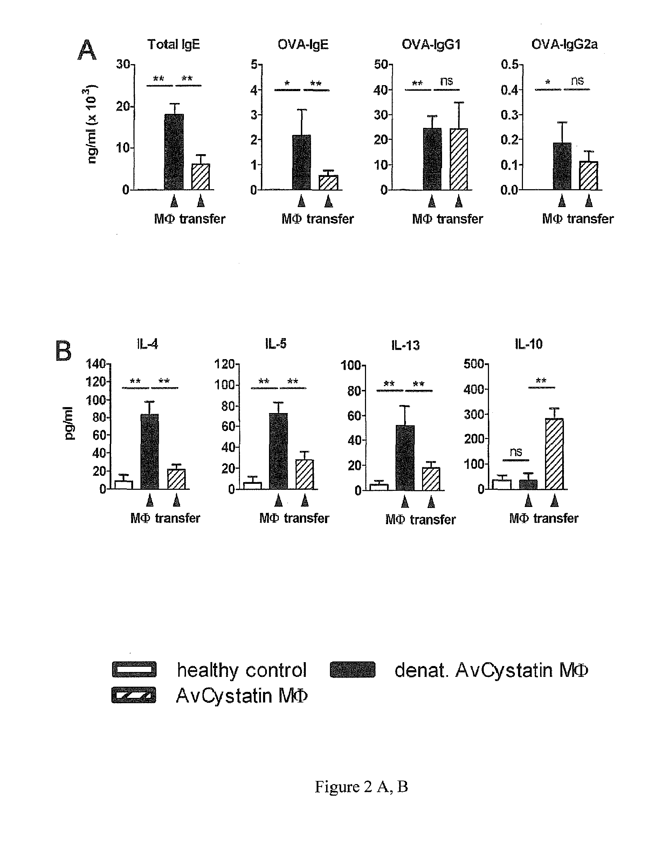

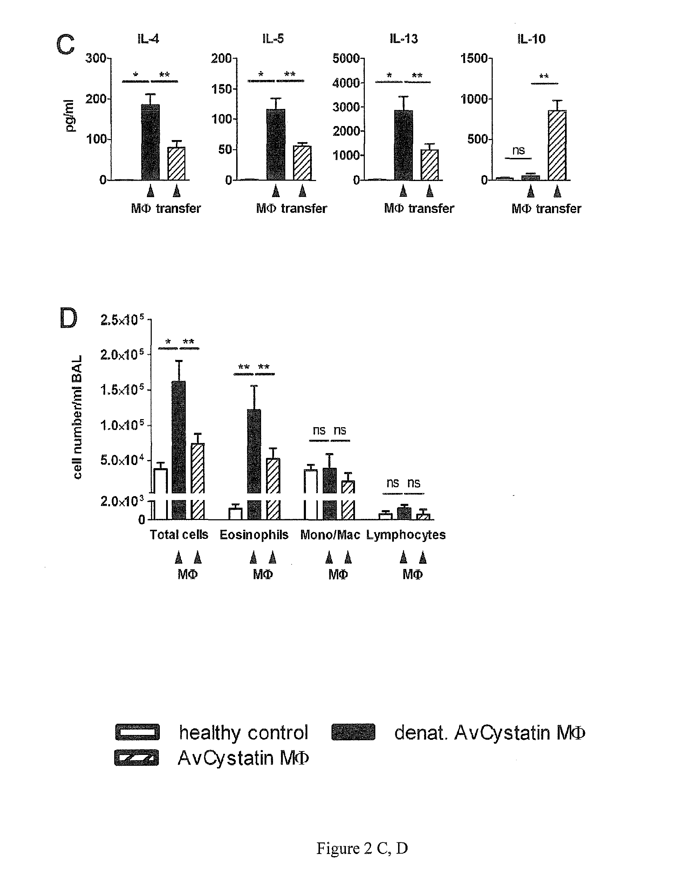

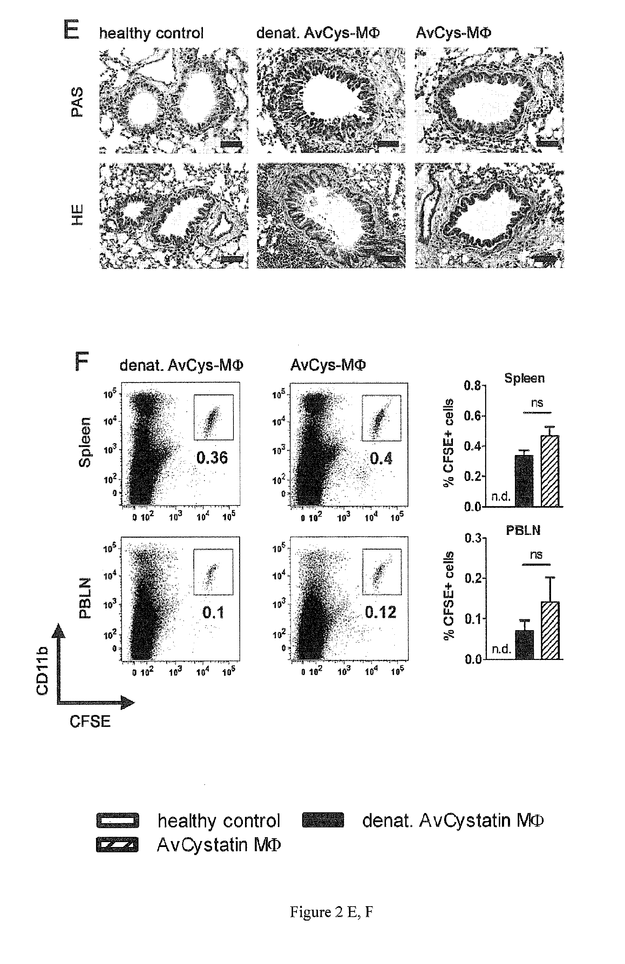

Figure 2 shows that macrophages ameliorate allergic inflammation in mice.

Macrophages were purified from PEC of AvCystatin or control pre-treated mice 18 hours post injection and transferred in OVA-sensitized mice.

Panel A shows the levels of total and allergen-specific IgE, OVA-IgG1 and OVA-IgG2a in recipient mice.

Panel B shows the concentrations of IL-4, IL-5, IL-13 and IL-10 in BAL-fluid.

Panel C shows Th2 cytokine levels in culture supernatants of allergen restimulated splenocytes.

Panel D shows the result of an analysis of BAL-fluid for total cells, eosinophils, monocytes/macrophages and lymphocytes.

Panel E shows the result of a histological analysis of mucus production and inflammatory cell influx by PAS and HE staining. In the sample "AvCys-Mϕ", reduced levels of PAS and HE staining are observed around the lumen compared to the sample "denat. AvCys-Mϕ". Thus, the figure illustrates a reduced mucus production (PAS staining) and reduced infiltration of inflammatory cells (HE staining) into the airways of asthmatic mice (Ova model) after injection of AvCystatin macrophages from wild type mice. Transfer of AvCystatin primed macrophages into asthmatic mice is able to soften asthma symptoms. Bars, 100 µm.

Panel F shows detection of CFSE+ (carboxyfluorescein succinimidyl ester positive) macrophages in spleen and PBLN (peribronchial lymph nodes) by flow cytometry analysis on day 31.

Values represent means ± SEM from two independent experiments with 5-6 mice per group for panels A-E and 3 mice per group for panel F. *, P < 0.05; **, P < 0.01.

### Figure 3

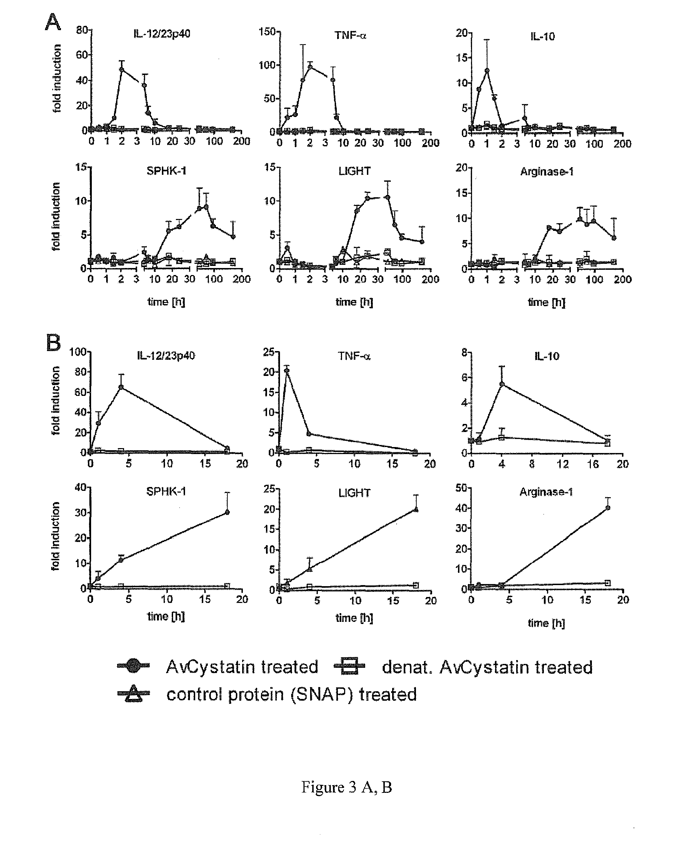

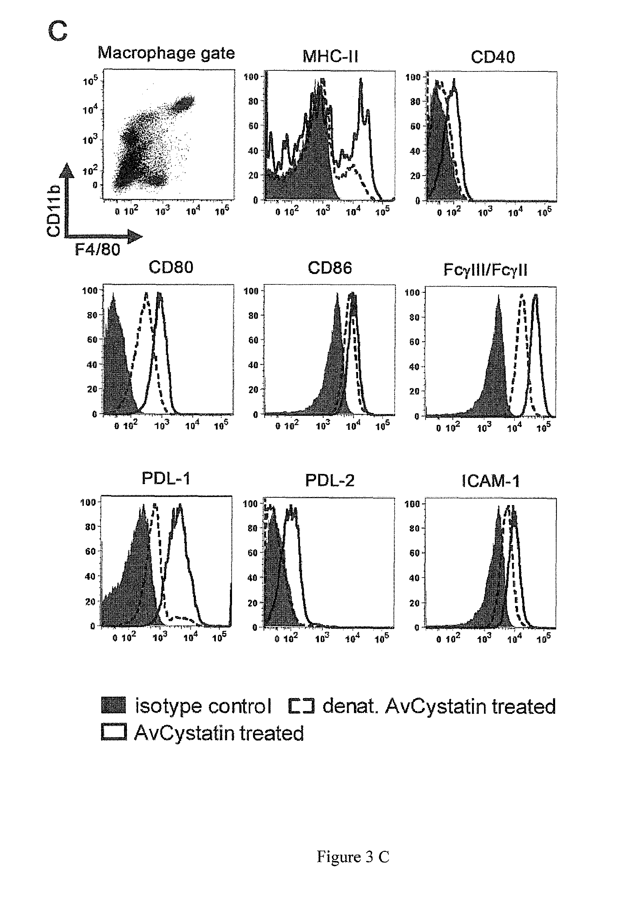

Figure 3 shows a phenotypic characterisation of AvCystatin elicited murine macrophages.

Macrophages were treated *in vitro* with AvCystatin or denatured AvCystatin and analysed for gene expression. In parallel, mice were intraperitoneally injected with 20 µg AvCystatin or control treatment, macrophages purified from isolated PEC and determined for gene expression.

Panel A shows the result of real-time PCR analyses for expression of selected murine macrophage marker genes *in vitro.* Results (mean ± SEM) are representative of three independent experiments.

Panel B shows the result of transcriptional profiling of macrophages isolated from AvCystatin or control pre-treated mice. Results (means ± SEM) are representative of two independent experiments. Normalized data are expressed as fold induction compared to untreated control mice. For each group, macrophages from 8-10 animals were pooled and gene expression analysed in triplicate values.

Panel C shows the result of a surface marker analysis for expression of MHC-II, co-stimulatory molecules CD40, CD80, CD86 and FcγIII/FeγII (CD16/32), PDL-1, PDL-2 and ICAM- 1 in F480+/CD11b+ peritoneal macrophages after i.p. (intraperitoneal) treatment of mice with AvCystatin or controls for 18h. Data from one representative of three independent experiments. Cells were pooled from 5-6 mice per group.

### Figure 4

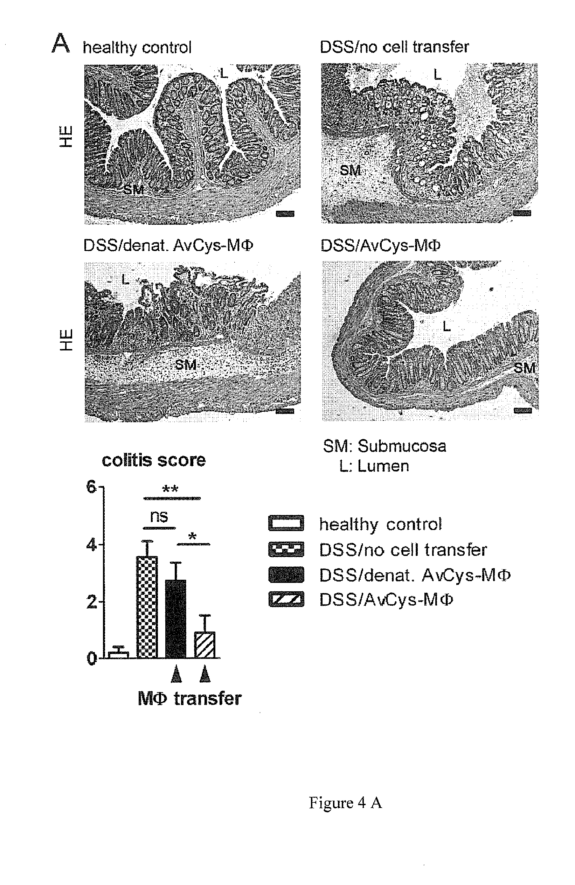

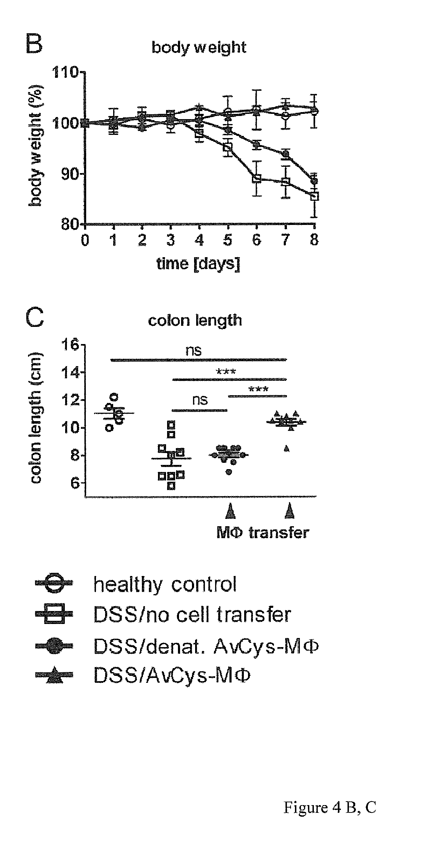

Figure 4 shows that AvCystatin derived regulatory macrophages transmit protective effects in DSS-induced colitis.

AvCystatin or control pre-treated macrophages were intravenously transferred in mice one day after access to DSS.

Panel A shows the result of a histological analysis of distal colon sections by HE staining and scoring. The figure shows HE staining of distal colon sections demonstrating less epithelial and mucosal damage as well as reduced influx of inflammatory cells after transfer of AvCystatin-treated macrophages compared to transfer of control-macrophages that shows no protective effects or DSS treatment without cell transfer.

Panel B shows the result of an analysis of changes in body weight.

Panel C shows the result of an analysis of changes in colon length.

Bars, 100 µm. Data represent means ± SEM from 2 experiments using 5-10 animals per group. *, P < 0.05; ***, P < 0.001

### Figure 5

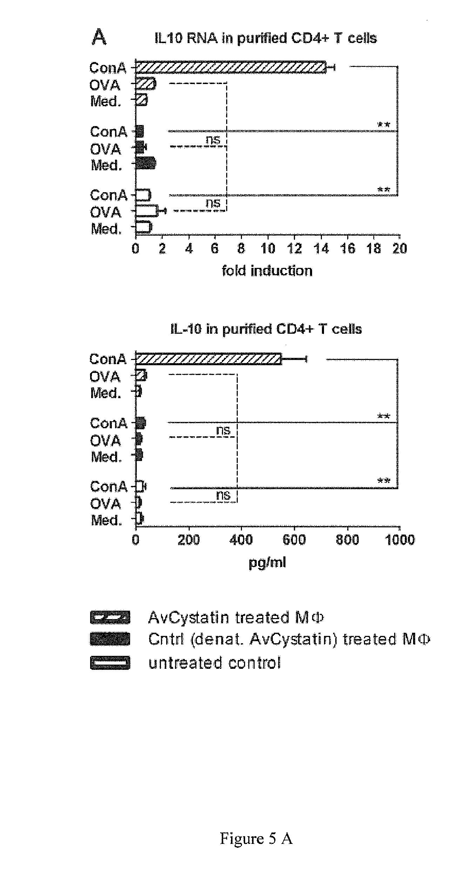

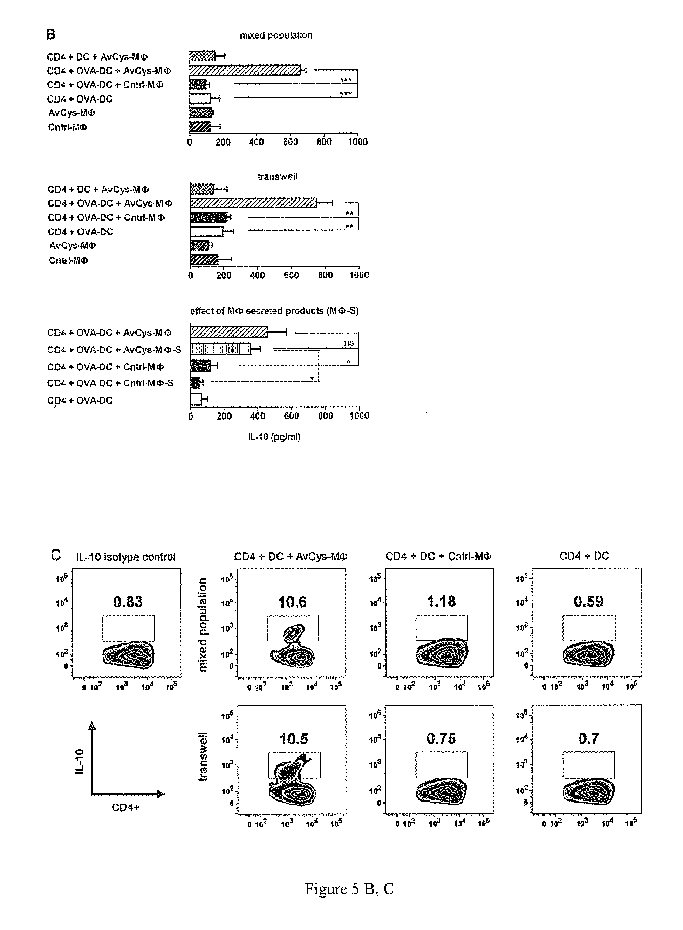

Figure 5 shows that AvCystatin-macrophages *in vivo* and *in vitro* induce IL-10-producing CD4+ T cells.

Panel A: CD4+ T cells were purified from the spleen of healthy mice or allergic animals after treatment with AvCystatin- or control-macrophages. IL-10 RNA (upper panel) and IL-10 protein (lower panel) was measured after restimulation with either ovalbumin or ConA (Concanavalin A) Results (mean ± SEM) are shown from one of two independent experiments with 3-6 mice per group. Normalized data are expressed as fold induction compared to healthy control mice. **, P < 0.01.

Panel B: Detection of IL-10 in culture supernatants after co-culture of *in vivo* generated AvCystatin or control-macrophages with ovalbumin-transgenic CD4+ T cells and ovalbumin peptide pre-treated DC (dendritic cells). Assays were performed in a mixed population and transwell approach or CD4+ T cells and DCs were alternatively incubated with macrophage supernatants (MΦ-S).

Panel C shows intracellular staining of IL-10 in CD4+ T cells after *in vitro* co-culture with DC and macrophages. Data is presented as mean ± SEM for one representative of at least three independent experiments. Triplicate analysis was performed for pooled cells from 3-6 mice.*,P<0.05;**,P<0.01;***,P<0.001.

### Figure 6

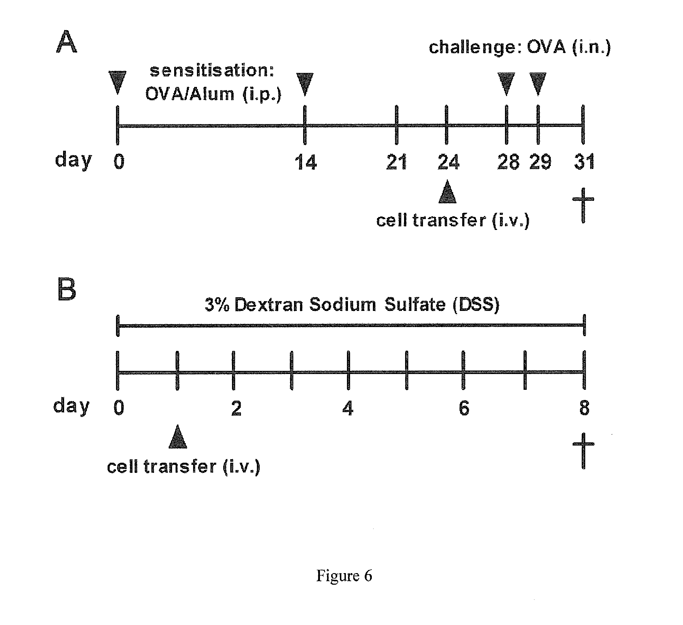

Figure 6 shows schemes of the disease models of ovalbumin-induced airway inflammation and DSS (dextran sulfate sodium)-induced colitis.

Panel A shows a scheme of the ovalbumin-induced airway inflammation disease model. Animals were intraperitoneally sensitized with 20 µg/animal OVA/Alum in PBS (phosphate buffered saline) on days 0 and 14 and intranasally challenged with 50 µg/animal ovalbumin on days 28 and 29.

Panel B shows a scheme of the DSS-induced colitis disease model. Animals were given 3% DSS in solution from day 0 to 8.

### Figure 7

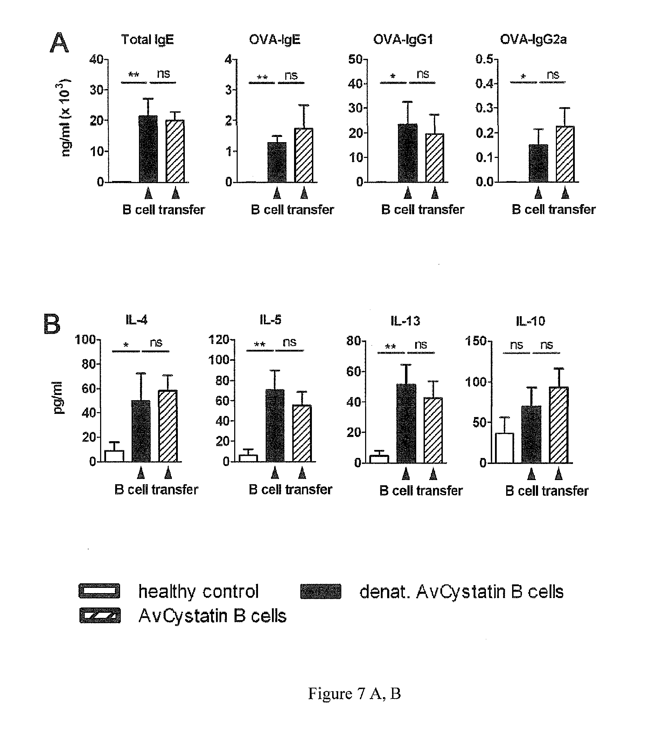

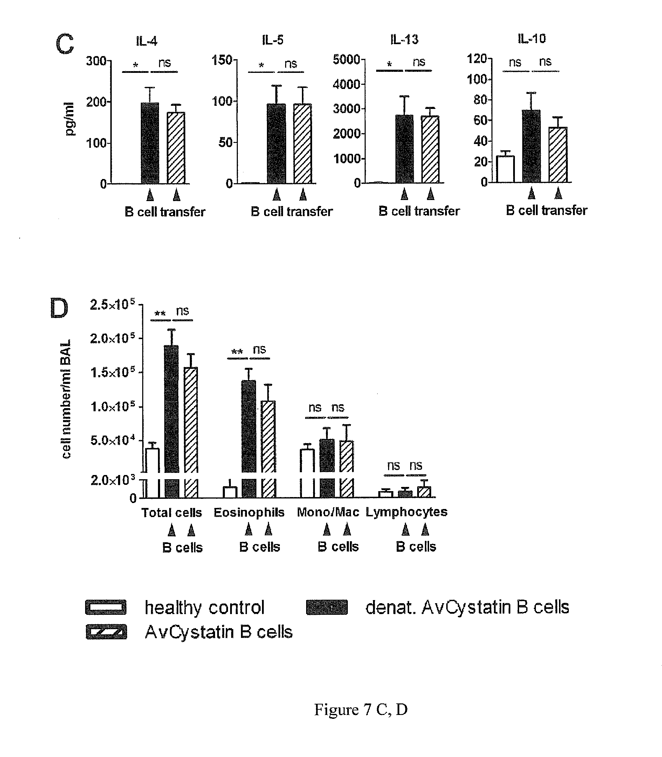

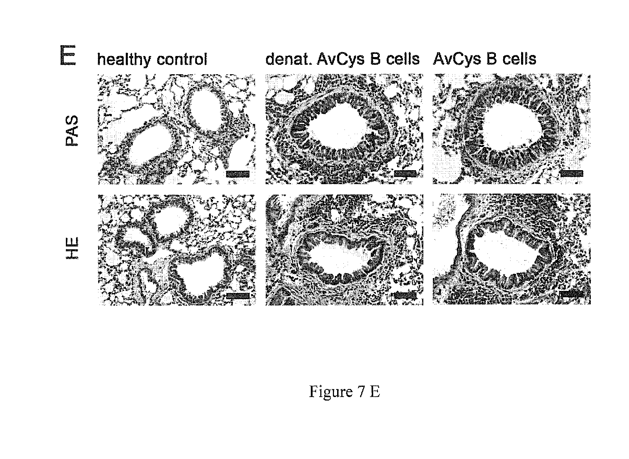

Figure 7 shows that transfer of B cells from AvCystatin treated animals does not protect against ovalbumin-induced airway inflammation.

Purified B cells from AvCystatin or control treated mice were transferred in OVA-sensitized mice 18 h post initial treatment.

Panel A shows total and allergen-specific IgE, OVA-IgG1 and OVA-IgG2a.

Panel B shows the Th2 cytokine response in BAL-fluid.

Panel C shows systemic cytokine levels in splenocyte culture supernatants after restimulation with ovalbumin.

Panel D shows inflammatory cells in BAL-fluid.

Panel E shows PAS and HE staining of lung sections. The levels of PAS and HE staining observed around the lumen are very similar between the samples "denat. AvCys B cells" and "AvCys B cells". Thus, the figure illustrates that transfer of B cells, isolated from AvCystatin primed pertoneal exudate cells, has no effect on mucus production (PAS staining) and infiltration of inflammatory cells (HE staining) into the airways of asthmatic mice (Ova model). AvCystatin primed B cells do not contribute to the amelioration of asthma symptoms in a mouse model of asthma. Bars, 100 µm.

Values represent means ± SEM from two independent experiments with 5-6 mice per group. *, P<0.05; **, P<0.01.

### Figure 8

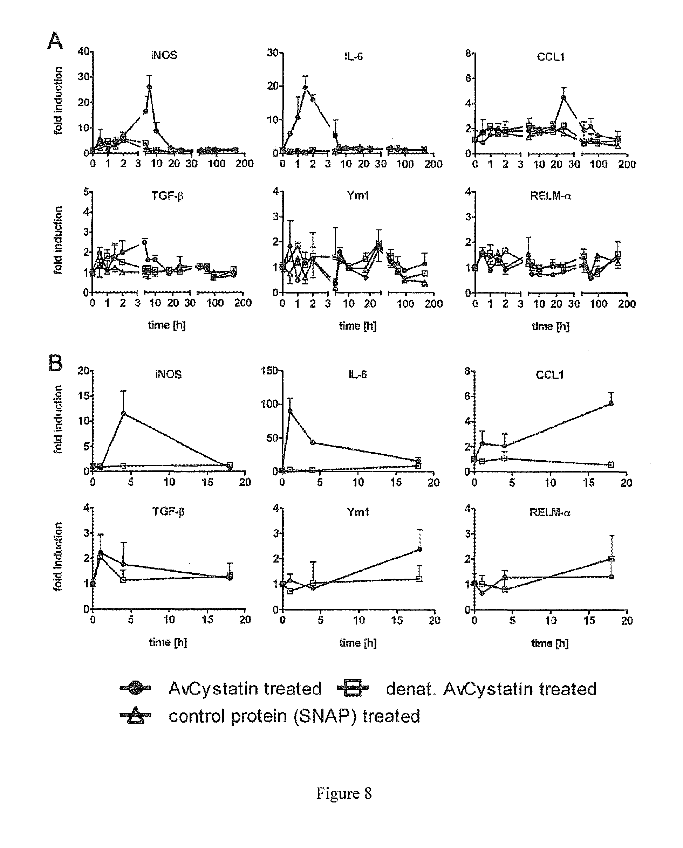

Figure 8 shows additional gene expression data on macrophage phenotyping (transcriptional profiling of further gene markers of AvCystatin elicited macrophages).

Panel A shows the result of real-time PCR analyses for expression of iNOS, IL-6, CCL1, TGF-β, Ym1 and RELM-α *in vitro.*

Panel B shows the result of real-time PCR analyses for expression of iNOS, IL-6, CCL1, TGF-β, Ym1 and RELM-α *ex vivo.*

Results (mean ± SEM) are representative of three (Panel A) and two (Panel B) independent experiments. Results are shown as means ± SEM. Normalized data are expressed as fold induction compared to untreated control mice. For each group, macrophages from 8-10 animals were pooled and gene expression analysed in triplicate values.

Moreover, reference is made to the following sequences, wherein SEQ ID NO:1 is the protein sequence of cystatin of *Acanthocheilonema viteae* (cystatin L43053), SEQ ID NO:2 is the protein sequence of cystatin of *Onchocerca volvulus* (cystatin M37105), and SEQ ID NO:3 is the protein sequence of cystatin of *Brugia malayi.*
SEQ ID NO:1 
L43053[Acanthocheilonema viteae]
MMLSIKEDGLLVVLLLSFGVTTVLVRCEEPANMESEVQAPNLLGGWQERNPEEKEIQDLLPKVLIKLNQL
SNVEYHLMPIKLLKVSSQVVAGLRYKMEIQVAQSECKKSSGEEVNLKTCKRLEGHPDQIITLEAWEKSWE
NFLQVKILEKKEVLSSV
SEQ ID NO:2 
M37105 [Onchocerca volvulus]
MLTIKDGTLLIHLLLFSVVALVQLQGAKSARAKNPSKMESKTGENQDRPVLLGGWEDRDPKDEEILELLP
SILMKVNEQSNDEYHLMPIKLLKVSSQVVAGVKYKMDVQVARSQCKKSSNEKVDLTKCKKLEGHPEKVMT
LEVWEKPWENFMRVEILGTKEV
SEQ ID NO:3

AF177193_1 [Brugia malayi]
MMSTMSIKEGLLVILLSLFLFDTTALIHRREIPHMESKGQMQRGQVLLGGWQERSPEDNEILELLPSVLT
KVNQQSNDEYHLMPIKLLKVSSQVVAGVKYKMEVQVARSECKKSASEQVNLKTCKKLEGHPDQVMTLEVW
EKPWEDFLQVNILETKVLSSV

In the following, reference is made to the examples which are given to illustrate, not to limit the present invention.

### Examples

### Example 1

### Production of Cystatin from Nematodes

The cDNAs of A. viteae-cystatin (Av17, Hartmann, S., Kyewsky, B., Sonnenburg, B. & Lucius, R. (1997) A filarial cysteine protease inhibitor downregulates T cell proliferation and enhances IL-10 production. Eur. J. Immunol. 27, 2253 - 2260.), and of *O*. *volvulus*-cystatin (Ov17, Lustigman S, Brotman B, Huima T, Prince AM, McKerrow JH. (1992) Molecular cloning and characterization of onchocystatin, a cysteine proteinase inhibitor of Onchocerca volvulus. J Biol Chem. 267:17339-46.); Schönemeyer, A., Lucius, R., Sonnenburg, B., Brattig, N., Sabat, R., Schilling, K., Bradley, J. & Hartmann, S. (2001) Modulation of human T cell responses and macrophage functions by onchocystatin, a secreted protein of the filarial nematode Onchocerca volvulus. J. Immunol. 167: 3207 - 3215) without the sequence encoding for the signal peptide were amplified by PCR using primers (Ov17: forward primer: 5'-gttcagttgcaaggagcc-3' (SEQ ID NO: 4), reverse primer: 5'tcatacttcttttgttccc3' (SEQ ID NO: 5); Av17: forward primer: 5'gttttggtgcgctgtgaa3' (SEQ ID NO: 6), reverse primer: 5'-tcacactgatgagagtac-3' (SEQ ID NO: 7)) derived from the full-length sequences. The PCR fragments were cloned into a T-overhang vector (pGEM-T Easy Vector Systems; Promega, Madison, WI) and further subcloned into the Eco-RI site of an expression vector yielding polypeptides with a leader of 6 histidines (pET-28 System; Novagen, Madison, USA). The plasmids were transformed into competent *E. coli* BL21 cells.

### Protein expression and purification

Screening of transformants for expression was carried out by analysis of bacterial protein after induction with isopropylgalactoside (IPTG). The cell pellet of a 1.5 ml culture was analysed by SDS-PAGE, followed by staining with Coomassie blue. The recombinant protein was purified from the E. coli lysate using a glycerol-PBS-buffer (phosphate buffer saline (PBS) with 10% glycerol) by means of affinity chromatography using a nickel-NTA-column, and subsequently eluted from the column by a pH-change (from pH6 to pH5 to pH3). Subsequently, the eluted protein was dialyzed against PBS/0.05% Triton and sterile filtered.

Recombinant AvCystatin and control protein (SNAP-tag protein, New England Biolabs) were expressed in *E. coli* and purified by affinity chromatography as described above. Endotoxin levels for purified proteins were below 0.1 endotoxin units/ml (EU/ml). To exclude that AvCystatin modulating effects on macrophages arise from contaminations in the buffer an additional control was included by using denatured AvCystatin (heat inactivated AvCystatin) with similar residual LPS (lipopolysaccharide) as AvCystatin protein.

### Animals

If not otherwise stated animals were purchased from Charles River or the Federal Institute for Risk Assessement (BfR) in Berlin.

### Model of ovalbumin-induced airway inflammation

BALB/c mice were sensitized upon intraperitoneal injections on days 0 and 14 with 20 µg ovalbumin (Sigma-Aldrich) emulsified in 2 mg aluminum hydroxide (Inject Alum, Pierce) in 0.2 ml PBS. Airway challenge was performed with intranasal application of 50 µg ovalbumin per animal on days 28 and 29. Naïve control mice were sensitized with aluminum hydroxide in PBS and challenged with PBS instead of ovalbumin. On day 31 animals were sacrificed. Blood samples were collected for serum antibody analysis and the trachea flushed with a protease inhibitor cocktail (Roche completeTM Mini protease inhibitor tablets dissolved in PBS) for cytokine detection. Cytospin slides were prepared from 100 µl BAL fluid, stained with the Hemacolor® staining set for microscopy (Merck) and analysed in a blinded fashion to determine cell numbers. Moreover, lungs were fixed in 3.5% Formalin for histological analysis of mucus production in bronchioles (PAS staining) and inflammatory cell infiltration in lung tissue (HE staining). Stained slices were analyzed via light microscopy (Leica DFC480 camera and Leica Application Suite Software version 2.8.1). Splenocytes from recipient mice were restimulated four days in 96 well plates with 50 µg/ml OVA or 2.5 µg/ml ConA in RPMI supplemented with 10% FCS (fetal calf serum), 1 mM LGlutamin, 100 U/ml Penicillin G and 100 µg/ml Streptomycin (cRPMI). Supernatants were frozen at - 20 °C for cytokine measurement. Cytokine levels were assessed for IL-4, IL-5, IL-13 and IL-10 by sandwich ELISA according to the manufacturer's protocol (eBioscience). For detection of OVAspecific IgE, ELISA plates were coated with 10 µg/ml ovalbumin at 4°C overnight and unspecific binding blocked by 3% BSA (bovine serum albumin) in PBS for 1 h. After incubation with mouse serum, HRP(horseradish peroxidase)-IgE (rat antimouse IgE heavy chain HRP (MCA419P), Serotec) was used as detection antibody and specific binding visualized by application of the HRP-substrate TMB (Tetramethylbenzidine). OVA-specific IgG1 and IgG2 were measured in an analogical manner whereas alkaline phosphatase conjucated antibodies (anti-mouse IgG1 (Gamma 1 chain) and anti-mouse IgG2a (Gamma 2a chain), both Rockland Immunochemicals) were used as detection antibodies and nitrophenylphosphat (Sigma-Aldrich) as phosphatase substrate. To calculate antibody amounts, purchasable IgE- (purified mouse IgE, κ isotype control, BD Biosciences), IgGl-(purified mouse IgG1, κ isotype control, BD Biosciences) and IgG2a- (purified mouse IgG2a, κ isotype control, BD Biosciences) standards were used. To determine total IgE levels, plates were coated with 4 µg/ml IgE capture antibody (purified anti-mouse IgE (R35-72), BD Biosciences) at 4°C overnight and further treated as described above for OVA specific IgE.

### Cell purification and adoptive transfer

Mice were intraperitoneally treated with 20 µg recombinant AvCystatin or control treatment. 18 hours later, peritoneal exudate cells (PEC) were isolated and either directly used for cell transfer (2x10⁶) or further purified for macrophages and B cells. Purification of B cells was performed by incubating PEC with anti-CD19 MicroBeads (Miltenyi Biotec) and cell separation upon autoMACSTM. Macrophages were purified by incubating PEC with biotinylated antibodies anti-CD3ε (clone 145-2C11, Miltenyi Biotec), anti-CD19 (clone eBiolD3, eBioscience), anti-CD11c (clone N418, eBioscience), anti-Gr1 (clone RB6-8C5, kind gift of the DRFZ) and anti-Dx5 (clone Dx5, eBioscience). Addition of 40 µg/ml anti-mouse FcγR (clone 2.4G2, DRFZ) was used to avoid non-specific binding. Cells were washed with PBS supplemented with 0.2% BSA and 2 mM EDTA (ethylenediaminetetraacetic acid) and incubated with anti-Biotin MicroBeads (Miltenyi Biotec) as described in the manufacturer's protocol. After incubation, magnetic cell separation was performed using autoMACS (Miltenyi Biotec). Purity of macrophages (>95 %) or B cells (90-95%) was determined by flow cytometry and cytospin analyses. 2x10⁶ purified macrophages or B cells were resuspended in 0.2 ml PBS and intravenously transferred on day 24 in ovalbumin sensitized mice prior to intranasal provocation. To examine macrophage tracking, purified macrophages were labelled with 5 µM CFSE for 8 minutes at room temperature. Labelling was stopped by adding FCS, cells were washed with PBS and adoptively transferred in recipient mice. Seven days post transfer BAL fluid, lung tissue, splenocytes and peribronchial lymph nodes were analyzed for CFSE positive macrophages by flow cytometry.

### Macrophage characterization

For *in vitro* characterization PEC were seeded in plastic tissue-culture dishes (24-well plates) and incubated at 37°C and 5% CO₂ in Dulbecco's modified Eagle's Medium (DMEM) supplemented with 10% fetal bovine serum, 100 U/ml Penicillin G and 100 µg/ml Streptomycin (cDMEM). After 4 hours, non adhering cells were removed by washing with fresh cDMEM. Remaining macrophages were cultured over night in cDMEM and stimulated on the following day with either 0.5 µM recombinant AvCystatin or control treatments. Macrophages were washed with Dulbecco`s PBS after indicated timepoints and frozen at - 80°C in lysis buffer (analytikjena). For *ex vivo* characterization macrophages were purified from isolated PEC of AvCystatin or control treated animals 18 hours post application as described above. Purified macrophages were lysated and frozen at -80°C. Surface marker expression was determined for CD11b+F4/80+ macrophages 18 h post i.p. application of AvCystatin or control treatment by using anti-MHC-II-FITC (clone M5/114.15.2, eBioscience), anti-CD274-PE (clone MIH5, BD Biosciences), anti-CD273-PE (clone TY25, BD Biosciences), anti-CD40-PE (clone 1C10, eBioscience), anti-CD86-biotin (clone GL1, eBioscience), streptavidin-PE-Cy7 (clone Streptavidin, BD Biosciences), anti-CD16/32-APC (clone 2.4G2, kind gift from the DRFZ), anti-CD80-PE (clone 16-10A1, eBioscience) and anti-ICAM-1-APC (clone KAT1, kind gift from the DRFZ). Non-specific binding was prevented by the addition of 20 µg/ml anti-mouse FcγR (clone 2.4G2, kind from the DRZF). After antibody binding cells were washed with Dulbecco's PBS supplemented with 0.2% BSA and 10 mM EDTA and flow cytometry analysis performed by using FACS-Canto cytometer (BD) and FlowJo Software version 8.5.3.

### RNA isolation, reverse transcription and real-time PCR

Total RNA isolation and reverse transcription was performed by using the innuPREP RNA kit (Analytik Jena) and the High Capacity RNA-to-cDNA Kit (Applied Biosystems) according to the manufacturer's instructions. Real-time PCR was performed with the ABI Prism 7300 Sequence Detection System (Applied Biosystems) using SYBR Green PCR reagents (FastStart Universal SYBR Green Master (Rox), Roche) following the manufacturer's guidelines. Relative changes in gene expression were calculated with ABI 7300 SDS software (Applied Biosystems) and expression levels of transcripts normalized to the ct values of the endogenous housekeeping gene Peptidylprolyl isomerase A (PPIA) by using the 2^{-ΔΔct} method (Livak and Schmittgen, 2001). In order to analyze marker gene expression in murine macrophages the following primer sequences were used:
SPHK-1 (NM_011451) fw: GGCAGTCATGTCCGGTGATG (SEQ ID NO: 8),
SPHK-1 (NM_011451) rv: ACAGCAGTGTGCAGTTGATGA (SEQ ID NO: 9);
LIGHT (NM_019418) fw: CTGCATCAACGTCTTGGAGA (SEQ ID NO: 10),
LIGHT (NM_019418) rv: GATACGTCAAGCCCCTCAAG (SEQ ID NO: 11);
Arginase-1 (NM_007482) fw: CAGAAGAATGGAAGAGTCAG (SEQ ID NO: 12),
Arginase-1 (NM_007482) rv: CAGATATGCAGGGAGTCACC (SEQ ID NO: 13);
Ym1 (NM_009892) fw: CATGAGCAAGACTTGCGTGAC (SEQ ID NO: 14),
Ym1 (NM_009892) rv: GGTCCAAACTTCCATCCTCCA (SEQ ID NO: 15);
RELM-α (NM_020509) fw: TCCCAGTGAATACTGATGAGA (SEQ ID NO: 16),
RELM-α (NM_020509) rv: CCACTCTGGATCTCCCAAGA (SEQ ID NO: 17);
PPIA (NM_008907) fw: GAGCTGTTTGCAGACAAAGTTC (SEQ ID NO: 18),
PPIA (NM_008907) rv: CCCTGGCACATGAATCCTGG (SEQ ID NO: 19).

### IL-10 expression by T cells

CD4+ were purified from spleens of ovalbumin-sensitized and -challenged mice seven days after adoptive transfer of macrophages. CD4+ T cells were isolated by using the CD4+ T cell isolation kit according to the manufacturer's guidelines (Miltenyi Biotec, Bergisch Gladbach, Germany). 3x10⁶ cells were stimulated in a 96 well plate (Coming Costar, Bodenheim, Germany) with ovalbumin (50 µg/ml) or ConA (2.5 µg/ml) for 96 hours in cRPMI. Supernatants were collected and frozen for IL-10 cytokine ELISA (BD Biosciences, Heidelberg, Germany). Cells were treated with lysis buffer for RNA extraction (Analytikjena, Jena, Germany), RNA isolated from cell lysates, transcribed into cDNA and IL-10 expression determined by quantitative real time-PCR.

In order to generate dendritic cells from BALB/c mice, the bone marrow was flushed with RPMI supplemented with 100 U/ml Penicillin G and 100 mg/ml Streptomycin from the femur and tibia of hind legs and collected in a pre-cooled sterile petri dish (Sarstedt, Nümbrecht, Germany). The marrow was dissociated by slightly pipetting up and down and cells filtered through a 70 µM nylon cell strainer (BD Biosciences, Heidelberg, Germany). After centrifugation (1200 rpm, 4°C, 10 min), erythrocyte lysis was performed for 5 minutes on ice. Cells were washed, counted, seeded into a petri dish with a density of 4x10⁶ cells in 10 ml cRPMI supplemented with 20 ng/ml murine GM-CSF (PreproTech, Hamburg, Germany) and incubated at 37°C and 5% CO₂. Three days later, 10 ml pre-warmed cRPMI containing 10 ng/ml GM-CSF was added to the cells. DCs were harvested on day seven, resuspended in fresh cRPMI and either directly used for co-culture assays or pre-treated with 1 µg/ml OVA-peptide 323-339 (GenScript, Piscataway, USA). After incubation at 37°C for two hours, the remaining peptide was washed away and DCs were used for co-culture experiments. Splenocytes were prepared from the spleen of euthanized BALB/c DO11.10 TGN mice and CD4+ T cells purified by using the CD4+ T cell isolation kit according to the manufacturer's description (Miltenyi Biotec, Bergisch Gladbach, Germany). Purification of macrophages from filarial cystatin (20 µg/mouse) or control-treated BALB/c mice was performed as described earlier on.

For co-culture assays CD4+ T cells were incubated with macrophages and dendritic cells in a ratio of 5:1:1. The assays were performed in a mixed population and a transwell approach separating the macrophages from dendritic cells and T cells by 1.0 µm filter membranes (greiner bio-one, Frickenhausen, Germany). Moreover, CD4+ T cells and dendritic cells were additionally incubated in the presence of fresh macrophage supernatants. Therefore, macrophages were separately cultured for 48 hours and the complete supernatant subsequently added to CD4+ T cells and dendritic cells. Culture supernatants were finally collected and analyzed for IL-10 by ELISA (BD Biosciences, Heidelberg, Germany). Remaining cells were intracellularly stained for IL-10. Therefore, cells were stimulated with 1 µg/ml PMA/Ionomyocin (Sigma-Aldrich, Steinheim, Germany) for three hours in fresh cRPMI at 37°C and 5% CO₂. After half an hour of incubation Brefeldin A (Sigma-Aldrich, Steinheim, Germany) was added in a concentration of 1 µg/ml. Cells were washed with FACS buffer, stained with anti-CD4-PerCP-eF710 (clone RM4-5; eBioscience, Frankfurt am Main, Germany) and incubated for 15 minutes on ice. In addition, 20 µg/ml anti-mouse-FcγR (clone 2.4G2; DRFZ, Berlin, Germany) was added to prevent unspecific binding. After washing, cells were permeabilized with the Fixation/Permeabilization kit from BD Biosciences, Heidelberg, Germany according to the manufacturer's instructions, intracellularly stained with anti-IL-10-PE (clone JES5-16E3; eBiosciences, Frankfurt am Main, Germany) and analyzed by LSRFortessa flow cytometer (BD Biosciences, Heidelberg, Germany) and FlowJo software version 8.8.7 (Tree Star, Ashland, USA).

### DSS-induced colitis

Colitis was induced by providing 3% DSS (MP Biomedicals, Lot No. 6683K) solution to female CL57/BL6 mice for 8 days. 3 x 10⁶ purified macrophages from AvCystatin or control treated CL57/BL6 were administered i.v. on day 1 of access to DSS. Changes in body weight were determined by weighing mice during d0 and d8. After dissection on day 8 colon length was measured and parts of distal colon fixed in 3.5% paraformaldehyde to evaluate the histological damage score upon HE staining.

### Statistical analysis

Statistical analysis and graphical output of data was performed with GraphPad Prism, version 5.0a (GraphPad Software). Data are presented as means ± SEM. The data were first tested with one way or two way ANOVA test followed by the Bonferroni's multiple comparison test to retrieve statistical significance between test groups. Differences between individual groups were tested to be significant for p < 0.05 (*), p < 0.01 (**) or p < 0.001 (***).

### Example 2

### Adoptive transfer of AvCystatin-primed peritonel cells from wild type and IL-10 deficient mice transmits tolerogenic effects in vivo.

All methods mentioned in this example were carried out as described in Example 1.

For this experiment, a mouse model of ovalbumin-induced airway inflammation was used. Donor cells were isolated 18h after i.p.-treatment with AvCystatin or control injections with denatured AvCystatin or an unrelated control protein and transferred four days prior to challenge with ovalbumin in recipient mice (Fig. 6, A). Administration of AvCystatin-primed PEC led to drastically reduced OVA-specific and total IgE levels compared to control treatments. However, OVA-specific IgG1 and IgG2a responses were not altered by transfer of AvCystatin-treated PEC (Fig. 1, A). Cytokine responses in BAL and spleen showed a drastic reduction of Th2 cytokines IL-4, IL-5 and IL-13 in animals treated with AvCystatin modulated cells. In addition, these animals responded with significantly increased local and systemic IL-10 levels (Fig. 1, B and C). Analysis of the cellular composition in bronchioalveolar lavage fluid (BAL-fluid) revealed a significant reduction of eosinophil infiltration to the lungs by 50% after transfer of AvCystatin-primed PEC once (Fig. 1, D). Histological lung sections confirmed that treatment of mice with AvCystatin primed cells resulted in reduced mucus production in bronchioles and lower airway cell infiltration compared to control treatments (Fig. 1, E).

When AvCystatin-primed PEC from IL-10 deficient mice were transferred in parallel, a comparable reduction in analyzed parameters such as IgE levels, cytokine response, cell recruitment, mucus production was observed. However, IL-10 production was highly elevated in recipients ameliorated from the disease (Fig. 1, A-E).

### Example 3

### Regulators macrophages induced by AvCystatin protect mice from airway inflammation.

All methods mentioned in this example were carried out as described in Example 1.

AvCystatin-induced macrophages from the peritoneum of donor mice 18h after treatment with AvCystatin were sorted and transferred. Purified peritoneal B cells after AvCystatin treatment were used as an additional control.

A single transfer of AvCystatin-induced macrophages (2x10⁶/mouse) 4 days prior to challenge with the allergen reduced allergic hyperreactivity in recipient mice. OVAspecific and total-IgE antibody responses were significantly suppressed in mice receiving the AvCystatin-induced regulatory macrophages compared to control treated macrophages (Fig. 2, A) or B cells from AvCystatin treated animals that showed no effect (Fig. 7). Th2-cytokines in BAL and spleen were hampered but IL- 10 levels significantly increased (Fig. 2, B and C). Eosinophil infiltration to the lungs was suppressed by 43% upon treatment with AvCystatin-induced regulatory macrophages (Fig. 2, D). However, OVA-specific IgG1 and IgG2a were not modified by the treatment with AvCystatin-induced macrophages (Fig. 2, A). Moreover, no induction of IFN-γ was determined (data not shown).

Next the localization of AvCystatin-induced regulatory macrophages within the recipient mice were examined. Thus, CFSE labelled cells were transferred intravenously 4 days prior to challenge with the allergen and their distribution in different tissues was analyzed by flow cytometry. CFSE labelled macrophages were recovered mainly in the spleen but were also detected to a lower amount in peribronchial lymph nodes (Fig. 2, F).

### Example 4

### Characterization of regulatory phenotype of macrophages induced by AvCystatin

All methods mentioned in this example were carried out as described in Example 1.

To characterize the macrophage phenotype induced by AvCystatin treatment marker genes of the four common macrophage phenotypes were analyzed: M1, M2a, M2b and M2c (Mantovani et al., 2004; Martinez et al., 2008). The AvCystatin-induced macrophages were at first characterized *in vitro.* AvCystatin treatment induced early expression of pro- and anti-inflammatory genes IL-12/23p40, TNF-α, iNOS, IL-6 and IL-10 (Fig. 3, A and 8, A). After 10-18h the expression of these marker genes decreased to background levels and switched from 18h towards a stable M2b-like profile with elevated levels of sphingosine-kinase-1 (SPHK1) and LIGHT (Fig. 3, A). In parallel, the M2a macrophage marker Arginase-1 (ARG1) was expressed. However, none of the other prominent M2a marker genes such as Ym1 or RELM-α were expressed (Fig. 8, A).

Next, the macrophage phenotype was examined *in* vivo after i.p. application of AvCystatin in BALB/c mice. Marker expression of purified macrophages revealed an identical expression pattern as determined *in vitro* (Fig. 3, B and 8, B). In conclusion, AvCystatin treatment stimulated a specific expression profile in macrophages with an early and transient induction of pro- and anti-inflammatory genes followed by a switch to a macrophage phenotype with a specific, novel expression profile.

Thereafter, the expression of macrophage cell surface markers was analyzed. 18h after AvCystatin application, F4/80+CD11b+ peritoneal macrophages were harvested, stained and analyzed by flow cytometry. Thereby, AvCystatin-elicited macrophages showed an up-regulation of MHC-II, CD40, CD80, FcγIII/FcγII (CD16/32), PDL-1 (CD274), PDL-2 (CD273), ICAM-1 and low levels of CD86 (Fig. 3, C).

Taken together, these data indicate that AvCystatin-derived macrophages reveal a distinct expression profile forcing a regulatory function.

### Example 5

### AvCystatin-induced regulatory macrophages prevent inflammation also in colitis

All methods mentioned in this example were carried out as described in Example 1.

The present inventors investigated whether AvCystatin-induced macrophages had any effects on an experimental model of colitis in mice. One day after starting DSS application to recipient mice 3x10⁶ AvCystatin-elicited macrophages were adoptively transferred. Treatment with AvCystatin-primed macrophages once led to an amelioration of colitis as measured by histological scoring of colon sections (Fig. 4, A), significant differences in body weight loss (Fig. 4, B) as well as in colon length (Fig. 4, C).

### Example 6

### Mechanistic analyses of IL-10 induction by AvCystatin-primed regulatory macrophages in vivo and in vitro

All methods mentioned in this example were carried out as described in Example 1.

CD4+ T cells (purity ≥ 95%) were purified from the spleens of healthy control mice or ovalbumin-sensitized and -challenged animals treated with denatured AvCystatin- or AvCystatin-macrophages prior to intranasal provocation. After restimulation of enriched CD4+ T cells with antigen (ovalbumin) or mitogen (ConA) levels of IL-10 protein were analyzed in CD4+ T cell culture supernatants and expression of IL-10 RNA in cell lysates of the stimulated CD4+ T cells. Thereby, a significant increase of IL-10 transcript levels (Fig. 5, A upper panel) and IL-10 protein (Fig. 5, A lower panel) was determined after polyclonal stimulation with ConA. The ovalbumin-induced IL-10 expression of CD4+ splenic T cells, however, was undetectable in comparison to the mitogen-induced IL-10 (Fig. 5, A). To verify the induction of IL-10-producing CD4+ T cells by AvCystatin-macrophages *in vitro,* we co-cultured AvCystatin- or control-treated macrophages with ovalbumin-transgenic CD4+ T cells from DO11.10 mice and bone marrow-derived dendritic cells pre-treated with ovalbumin-peptide 323-339 (OVA-DC) for sufficient T cell activation. To investigate whether IL-10 induction in CD4+ T cells is cell contact-dependent or mediated through the release of soluble factors, *in vitro* assays were performed in a mixed population and transwell approach separating the macrophages from T cells. In both cases co-culture with filarial cystatin-treated macrophages clearly induced IL-10 in CD4+ T cells whereas control-treated macrophages lacked this IL-10-inducing capacity (Fig. 5, B upper and middle panel). Moreover, CD4+ T cells and DC co-cultures in the presence of supernatants from AvCystatin- but not control-treated macrophages significantly increased levels of IL-10 in the culture supernatants (Fig. 5, B lower panel) favouring a cell contact-independent mechanism for the observed IL-10 induction. Co-culture of CD4+ T cells and macrophages with untreated dendritic cells did not induce IL-10 in T cells demonstrating that antigen-mediated CD4+ T cell activation is necessary for the induction of IL-10 (Fig. 5, B upper and middle panel). Intracellular staining of IL-10 verified CD4+ T cells to be the cellular origin for this cytokine in both the mixed population and transwell approach (Fig. 5, C).

The features of the present invention disclosed in the specification, the claims, and/or in the accompanying drawings may, both separately and in any combination thereof, be material for realizing the invention in various forms thereof.

### References

Anderson, C. A., and D.M. Mosser (2002). A novel phenotype for activated macrophages: the type II activated macrophage. J. Leukoc. Biol. 72: 101-106
Anthony, R.M., J.F. Urban, Jr, F. Alem, H.A. Hamed, C.T. Rozo, J.L. Boucher, N. Van Rooijen, and W.C. Gause (2006). Memory TH2 cells induce alternatively activated macrophages to mediate protection against nematode parasites. Nat Med. 12(8): 955-960
Bach JF (2002). The effect of infections on susceptibility to autoimmune and allergic diseases. N Engl J Med. 347(12): 911-20
Croese J, O'neil J, Masson J, Cooke S, Melrose W, Pritchard D, Speare R (2006). A proof of concept study establishing Necator americanus in Crohn's patients and reservoir donors. Gut. 55(1): 136-37
Davies JQ, Gordon S (2005 a). Isolation and culture of murine macrophages. Methods Mol Biol. 290: 91-103
Davies JQ, Gordon S (2005 b). Isolation and culture of human macrophages. Methods Mol Biol. 290: 105-16
Edwards, J.P., X. Zhang, K.A. Frauwirth, and D.M. Mosser (2006). Biochemical and functional characterization of three activated macrophage populations. J Leukoc Biol. 80(6): 1298-307
Fallon PG, Mangan NE (2007). Suppression of TH2-type allergic reactions by helminth infection. Nat Rev Immunol. 7(3): 220-30
Gordon, S. (2003). Alternative activation of macrophages. Nat Rev Immunol. 3(1): 23-35 Hawrylowicz CM, O'Garra A (2005). Potential role of interleukin-10-secreting regulatory T cells in allergy and asthma. Nat Rev Immunol. 5(4): 271-283
Jenkins SJ, Allen JE (2010). Similarity and diversity in macrophage activation by nematodes, trematodes, and cestodes. J Biomed Biotechnol. 2010, article 262609
Jenkins SJ, Ruckerl D, Cook PC, Jones LH, Finkelman FD, van Rooijen N, MacDonald AS, Allen JE (2011). Local macrophage proliferation, rather than recruitment from the blood, is a signature of TH2 inflammation. Science. 332(6035): 1284-88
Klotz, C., T. Ziegler, A.S. Figueiredo, S. Rausch, M.R. Hepworth, N. Obsivac, C. Sers, R. Lang, P. Hammerstein, R. Lucius, S. Hartmann (2011). A Helminth Immunomodulator Exploits Host Signaling Events to Regulate Cytokine Production in Macrophages. PLoS Pathogens. 7(1): e1001248
Kruisbeek AM (2001). Isolation of mouse mononuclear cells. Curr Protoc Immunol. Chapter 3: Unit 3.1
Livak KJ, Schmittgen TD (2001). Analysis of relative gene expression data using real-time quantitative PCR and the 2(-Delta Delta C(T)) Method. Methods. 25(4): 402-08
Mantovani, A., A. Sica, S. Sozzani, P. Allavena, A. Vecchi, and M. Locati (2004). The chemokine system in diverse forms of macrophage activation and polarization. Trends Immunol. 25(12): 677-86
Martinez, F.O., A. Sica, A. Mantovani, and M. Locati (2008). Macrophage activation and polarization. Front Biosci. 13: 453-61
Mills, C.D., K. Kincaid, J.M. Alt, M.J. Heilman, and A.M. Hill (2000). M-I/M-2 macrophages and the Th1/Th2 paradigm. J Immunol. 164(12): 6166-73
Mosser DM, Zhang X. (2008). Activation of murine macrophages. Curr Protoc Immunol. Chapter 14: Unit 14.2
Ray A, Dittel BN (2010). Isolation of mouse peritoneal cavity cells. J Vis Exp. 28;(35). pii: 1488. doi: 10.3791/1488
Schnoeller, C., S. Rausch, S. Pillai, A. Avagyan, B.M. Wittig, C. Loddenkemper, A. Hamann, E. Hamelmann, R. Lucius, and S. Hartmann (2008). A helminth immunomodulator reduces allergic and inflammatory responses by induction of IL-10-producing macrophages. J Immunol. 180(6): 4265-72
Senju S, M Haruta, K Matsumura, Y Matsunaga, S Fukushima, T Ikeda, K Takamatsu, A Irie and Y Nishimura (2011). Generation of dendritic cells and macrophages from human induced pluripotent stem cells aiming at cell therapy. Gene Therapy 18(9): 874-83
Smythies LE, Wahl LM, Smith PD (2006). Isolation and purification of human intestinal macrophages. Curr Protoc Immunol. 7: Unit 7.6B
Summers RW, Elliott DE, Urban JF Jr, Thompson RA, Weinstock JV (2005). Trichuris suis therapy for active ulcerative colitis: a randomized controlled trial. Gastroenterology. 128(4): 825-32
Wahl LM, Wahl SM, Smythies LE, Smith PD (2006). Isolation of human monocyte populations. Curr Protoc Immunol. Chapter 7: Unit 7.6A
Weischenfeldt J, Porse B (2008). Bone Marrow-Derived Macrophages (BMM): Isolation and Applications. CSH Protoc. 2008:pdb.prot5080. doi: 10.1101/pdb.prot5080
Yazdanbakhsh M, Kremsner PG, van Ree R (2002). Allergy, parasites, and the hygiene hypothesis. Science. 296(5567): 490-94
Zhang X, Goncalves R, Mosser DM (2008). The isolation and characterization of murine macrophages. Curr Protoc Immunol. Chapter 14: Unit 14.1

## Claims

1. A macrophage cell for use in the prevention and/or treatment of an inflammatory disease in a patient,
wherein, preferably, said cell has been produced by treatment with a cystatin derived from a parasitic nematode, and
wherein said cell differs from murine macrophages of subpopulation M2a by a higher expression level of the gene SPHK1 and/or the gene LIGHT, and from murine macrophages of subpopulation M2b by a higher expression level of the gene ARG1, as measured by real-time PCR.

2. The cell according to claim 1, wherein said macrophage cell is an autologous cell with respect to said patient.

3. The cell according to claim 1 or 2, wherein said macrophage cell is administered to said patient, preferably by intravenous administration.

4. An autologous macrophage cell for use in the prevention and/or treatment of an inflammatory disease in a patient, wherein said macrophage cell is prepared by the steps:
(a)
- obtaining a macrophage cell derived from the body of said patient,
- activating macrophage cell *in vitro* by treatment with a cystatin derived from a parasitic nematode, wherein, preferably, said cell is activated in the presence of bystander cells, preferably leukocytes,
or
(b)
- activating a macrophage cell in the body of said patient,
- obtaining said macrophage cell from the body of said patient.

5. The cell according to claim 4, wherein, after said macrophage cell has been prepared, it is administered to said patient, preferably by intravenous administration.

6. The cell according to claim 4 or 5, wherein said obtaining said cell derived from the body of said patient involves one of the following:
A) collecting blood from said patient and isolating macrophage precursors (monocytes) from said blood, preferably by differential adhesion to tissue culture plastic, optionally after removing components of the blood that are not cells, and differentiating said macrophage-precursors into macrophages in vitro by incubating said cells with macrophage colony-stimulating factor (M-CSF),
B) collecting blood of said patient and removing cell populations other than macrophage precursors from said blood, preferably removing T cells, B cells, granulocytes, dendritic cells, eosinophiles and/or NK cells, optionally after removing components of the blood that are not cells, and differentiating said macrophage-precursors into macrophages in vitro by incubating said cells with macrophage colony-stimulating factor (M-CSF),
wherein, preferably, said removing of cell populations other than macrophage precursors is achieved by cell depletion with antibodies binding to cell surface molecules of the respective cell types,
C) obtaining peritoneal exudate cells and, optionally, removing cell populations other than macrophages, preferably removing T cells, B cells, granulocytes, dendritic cells, eosinophiles and/or NK cells, preferably by cell depletion with antibodies binding to cell surface molecules of the respective cell types,
D) generating macrophages from induced human Pluripotent stem cells (iPS) of said patient.

7. The cell according to any of claims 4-6, wherein said cell differs from murine macrophages of subpopulation M2a by a higher expression level of the genes SPHK1 and/or LIGHT, as measured by real-time PCR, and/or wherein said cell differs from murine macrophages of subpopulation M2b by a higher expression level of the gene ARG1, as measured by real-time PCR.

8. The cell according to any of the preceding claims, wherein said cell differs from murine macrophages of subpopulation M2a by a lower expression level of the genes Ym1 and/or RELM-α, as measured by real-time PCR,
wherein, preferably, said lower expression level of Ym1 and/or RELM-α is, independently, at least 2-fold, preferably at least 5-fold, more preferably at least 10-fold, even more preferably at least 20-fold, most preferably at least 50-fold.

9. The cell according to any of claims 1-3 or 7-8, wherein said higher expression level of SPHK1, LIGHT, and/or ARG1 is, independently, at least 2-fold, preferably at least 5-fold, more preferably at least about 10-fold, more preferably at least about 20-fold, more preferably at least about 30-fold, more preferably at least about 40-fold, more preferably at least 50-fold, most preferably at least 100-fold.

10. The cell according to any of the preceding claims, wherein said inflammatory disease is an atopic and/or autoimmune disease,
preferably selected from the group comprising allergic diseases of the respiratory organs, such as asthma, hay fever, allergic sinusitis, allergic rhinitis, more preferably asthma or hay fever, allergic diseases of the gastrointestinal system, such as food allergies, allergic diseases of the skin, such as atopic dermatitis, systemic allergic diseases, such as anaphylactic reactions, allergic encephalomyelitis, autoimmune diseases of the joints and/or skin and/or internal organs, such as rheumatoid arthritis, psoriasis, lupus erythematosus, multiple sclerosis, and inflammatory bowel diseases, such as colitis ulcerosa and Crohn's Disease, more preferably colitis, even more preferably colitis ulcerosa.

11. The cell according to any of claims 3 or 5-10, wherein, after said cell is produced/prepared and before said cell is administered to said patient, said cell is stored in a frozen state.

12. The cell according to any of the preceding claims, wherein said cell is part of a mixture of cells with a content of macrophages higher than 85%, preferably higher than 90%, more preferably higher than 95%, most preferably higher than 98%.

13. The cell according to any of the preceding claims, wherein said cystatin has a sequence selected from the group comprising SEQ ID NO:1 (*Acanthocheilonema viteae* cystatin L43053), SEQ ID NO: 2 (*Onchocerca volvulus* cystatin M37105), SEQ ID NO:3 (*Brugia malayi* cystatin), and sequences that are 70% identical, preferably 80% identical, more preferably 90% identical and, most preferably, 99% identical to any of the foregoing.

14. The cell according to any of the preceding claims, wherein said cystatin is recombinant cystatin and has been produced by a prokaryotic or eukaryotic expression system.


SEQUENCE LISTING

<110>  Humboldt-Universität zu Berlin

<120>  Regulatory Macrophages and Their Uses

<130>  FB26376

<160>  19

<170>  PatentIn version 3.5

<210>  1
<211>  157
<212>  PRT
<213>  Acanthocheilonema viteae

<400>  1

Met Met Leu Ser Ile Lys Glu Asp Gly Leu Leu Val Val Leu Leu Leu
1               5                   10                  15

Ser Phe Gly Val Thr Thr Val Leu Val Arg Cys Glu Glu Pro Ala Asn
            20                  25                  30

Met Glu Ser Glu Val Gln Ala Pro Asn Leu Leu Gly Gly Trp Gln Glu
        35                  40                  45

Arg Asn Pro Glu Glu Lys Glu Ile Gln Asp Leu Leu Pro Lys Val Leu
    50                  55                  60

Ile Lys Leu Asn Gln Leu Ser Asn Val Glu Tyr His Leu Met Pro Ile
65                  70                  75                  80

Lys Leu Leu Lys Val Ser Ser Gln Val Val Ala Gly Leu Arg Tyr Lys
                85                  90                  95

Met Glu Ile Gln Val Ala Gln Ser Glu Cys Lys Lys Ser Ser Gly Glu
            100                 105                 110

Glu Val Asn Leu Lys Thr Cys Lys Arg Leu Glu Gly His Pro Asp Gln
        115                 120                 125

Ile Ile Thr Leu Glu Ala Trp Glu Lys Ser Trp Glu Asn Phe Leu Gln
    130                 135                 140

Val Lys Ile Leu Glu Lys Lys Glu Val Leu Ser Ser Val
145                 150                 155

<210>  2
<211>  162

<212>  PRT
<213>  Onchocerca volvulus

<400>  2

Met Leu Thr Ile Lys Asp Gly Thr Leu Leu Ile His Leu Leu Leu Phe
1               5                   10                  15

Ser Val Val Ala Leu Val Gln Leu Gln Gly Ala Lys Ser Ala Arg Ala
            20                  25                  30

Lys Asn Pro Ser Lys Met Glu Ser Lys Thr Gly Glu Asn Gln Asp Arg
        35                  40                  45

Pro Val Leu Leu Gly Gly Trp Glu Asp Arg Asp Pro Lys Asp Glu Glu
    50                  55                  60

Ile Leu Glu Leu Leu Pro Ser Ile Leu Met Lys Val Asn Glu Gln Ser
65                  70                  75                  80

Asn Asp Glu Tyr His Leu Met Pro Ile Lys Leu Leu Lys Val Ser Ser
                85                  90                  95

Gln Val Val Ala Gly Val Lys Tyr Lys Met Asp Val Gln Val Ala Arg
            100                 105                 110

Ser Gln Cys Lys Lys Ser Ser Asn Glu Lys Val Asp Leu Thr Lys Cys
        115                 120                 125

Lys Lys Leu Glu Gly His Pro Glu Lys Val Met Thr Leu Glu Val Trp
    130                 135                 140

Glu Lys Pro Trp Glu Asn Phe Met Arg Val Glu Ile Leu Gly Thr Lys
145                 150                 155                 160

Glu Val

<210>  3
<211>  161
<212>  PRT
<213>  Brugia malayi

<400>  3

Met Met Ser Thr Met Ser Ile Lys Glu Gly Leu Leu Val Ile Leu Leu
1               5                   10                  15

Ser Leu Phe Leu Phe Asp Thr Thr Ala Leu Ile His Arg Arg Glu Ile
            20                  25                  30

Pro His Met Glu Ser Lys Gly Gln Met Gln Arg Gly Gln Val Leu Leu

35                  40                  45

Gly Gly Trp Gln Glu Arg Ser Pro Glu Asp Asn Glu Ile Leu Glu Leu
    50                  55                  60

Leu Pro Ser Val Leu Thr Lys Val Asn Gln Gln Ser Asn Asp Glu Tyr
65                  70                  75                  80

His Leu Met Pro Ile Lys Leu Leu Lys Val Ser Ser Gln Val Val Ala
                85                  90                  95

Gly Val Lys Tyr Lys Met Glu Val Gln Val Ala Arg Ser Glu Cys Lys
            100                 105                 110

Lys Ser Ala Ser Glu Gln Val Asn Leu Lys Thr Cys Lys Lys Leu Glu
        115                 120                 125

Gly His Pro Asp Gln Val Met Thr Leu Glu Val Trp Glu Lys Pro Trp
    130                 135                 140

Glu Asp Phe Leu Gln Val Asn Ile Leu Glu Thr Lys Val Leu Ser Ser
145                 150                 155                 160

Val

<210>  4
<211>  18
<212>  DNA
<213>  Artificial Sequence

<220>
<223>  Ov17 forward primer

<400>  4
gttcagttgc aaggagcc                                                    18

<210>  5
<211>  19
<212>  DNA
<213>  Artificial Sequence

<220>
<223>  Ov17 reverse primer

<400>  5
tcatacttct tttgttccc                                                   19

<210>  6
<211>  18
<212>  DNA
<213>  Artificial Sequence

<220>

<223>  Av17 forward primer

<400>  6
gttttggtgc gctgtgaa                                                    18


<210>  7
<211>  18
<212>  DNA
<213>  Artificial Sequence

<220>
<223>  Av17 reverse primer

<400>  7
tcacactgat gagagtac                                                    18


<210>  8
<211>  20
<212>  DNA
<213>  Artificial Sequence

<220>
<223>  SPHK-1 (NM_011451) fw

<400>  8
ggcagtcatg tccggtgatg                                                  20


<210>  9
<211>  21
<212>  DNA
<213>  Artificial Sequence

<220>
<223>  SPHK-1 (NM_011451) rv

<400>  9
acagcagtgt gcagttgatg a                                                21


<210>  10
<211>  20
<212>  DNA
<213>  Artificial Sequence

<220>
<223>  LIGHT (NM_019418) fw

<400>  10
ctgcatcaac gtcttggaga                                                  20


<210>  11
<211>  20
<212>  DNA
<213>  Artificial Sequence

<220>
<223>  LIGHT (NM_019418) rv

<400>  11
gatacgtcaa gcccctcaag                                                  20

<210>  12
<211>  20
<212>  DNA
<213>  Artificial Sequence

<220>
<223>  Arginase-1 (NM_007482) fw

<400>  12
cagaagaatg gaagagtcag                                                    20


<210>  13
<211>  20
<212>  DNA
<213>  Artificial Sequence

<220>
<223>  Arginase-1 (NM_007482) rv

<400>  13
cagatatgca gggagtcacc                                                    20


<210>  14
<211>  21
<212>  DNA
<213>  Artificial Sequence

<220>
<223>  Ym1 (NM_009892) fw

<400>  14
catgagcaag acttgcgtga c                                                  21


<210>  15
<211>  21
<212>  DNA
<213>  Artificial Sequence

<220>
<223>  Ym1 (NM_009892) rv

<400>  15
ggtccaaact tccatcctcc a                                                  21


<210>  16
<211>  21
<212>  DNA
<213>  Artificial Sequence

<220>
<223>  RELM-alpha  (NM_020509) fw

<400>  16
tcccagtgaa tactgatgag a                                                  21


<210>  17
<211>  20
<212>  DNA
<213>  Artificial Sequence

<220>
<223>  RELM-alpha  (NM_020509) rv

<400>  17
ccactctgga tctcccaaga                                                   20


<210>  18
<211>  22
<212>  DNA
<213>  Artificial Sequence

<220>
<223>  PPIA (NM_008907) fw

<400>  18
gagctgtttg cagacaaagt tc                                                22


<210>  19
<211>  20
<212>  DNA
<213>  Artificial Sequence

<220>
<223>  PPIA (NM_008907) rv

<400>  19
ccctggcaca tgaatcctgg                                                   20



1



6